# EUROPEAN PATENT SPECIFICATION

(11) **EP 4 427 064 B1**
(45) Date of publication and mention of the grant of the patent: **22.04.2026**
(21) Application number: 22813172.8
(22) Date of filing: 28.10.2022
(51) Int. Cl.: G01R 33/567

(54) **RESPIRATORY STATE ALIGNMENT IN MRI**
AUSRICHTUNG DES ATMUNGSZUSTANDES IN DER MRT
ALIGNEMENT D'ÉTAT RESPIRATOIRE EN IRM

(30) Priority: 05.11.2021 EP 21206637
(43) Date of publication of application: 11.09.2024
(73) Proprietor: Koninklijke Philips N.V., 5656 AG Eindhoven (NL)
(72) Inventor: ALBERTS, Eveline, 5656 AG Eindhoven (NL); STEMKENS, Bjorn, 5656 AG Eindhoven (NL); HEUVELINK-MARCK, Annerieke, 5656 AG Eindhoven (NL)
(74) Representative: Philips Intellectual Property & Standards
(86) International application number: PCT/EP2022/080278
(87) International publication number: WO 2023/078804

(56) References cited:
- EP-A1- 3 805 773
- US-A1- 2013 251 225
- US-A1- 2018 188 344
- LIU YILIN ET AL: "Four dimensional magnetic resonance imaging with retrospectivek-space reordering: A feasibility study", MEDICAL PHYSICS, AIP, MELVILLE, NY, US, vol. 42, no. 2, 1 February 2015 (2015-02-01), pages 534 - 541, XP012193475, ISSN: 0094-2405, [retrieved on 19010101], DOI: 10.1118/1.4905044
- AKÇAKAYA MEHMET ET AL: "Free-breathing phase contrast MRI with near 100% respiratory navigator efficiency using k-space-dependent respiratory gating : Improved NAV Efficiency for 4D Flow", MAGNETIC RESONANCE IN MEDICINE, vol. 71, no. 6, 30 July 2013 (2013-07-30), US, pages 2172 - 2179, XP055911071, ISSN: 0740-3194, DOI: 10.1002/mrm.24874

## Description

### FIELD OF THE INVENTION

The present invention relates to the field of respiratory motion monitoring and image processing in magnetic resonance imaging. More specifically, the invention relates to a method, a magnetic resonance imaging system and computer-program product for respiratory monitoring of a subject's respiration during a magnetic resonance imaging session and taking this respiratory monitoring into account in controlling the data acquisition and/or processing the acquired data.

### BACKGROUND OF THE INVENTION

EP3805773A1 discloses a medical system that comprises a radiotherapy system configured for controllably irradiating a target volume within an irradiation zone; a subject support configured for supporting at least a ventral region of a subject within the irradiation zone; a breath monitor system configured for providing a motion signal, descriptive of subject breathing motion; and a subject display configured for displaying a breathing phase indicator to the subject when supported by the subject support.

US2018/0188344Al describes an MRI system for performing time resolved MR imaging of an object with grouped data acquisition is provided. The MRI system includes an MRI controller in electronic communication with a magnet assembly and operative to sample a group of data points within a first region of a k-space. The first region includes a central sub-region and a first peripheral sub-region. The MRI controller is further operative to sample a group of data points within a second region of the k-space. The second region includes the central sub-region and a second peripheral sub-region different from the first peripheral sub-region.

US2013/0251225Al describes a method that provides motion corrected MR image data in an MR imaging system. The method employs an imaging method for acquiring k-space data of a k-space data array during an imaging scan and the k-space data represents image data of a patient anatomical region. The method acquires k-space data associated with a central region of the k-space data array and subsequently acquires k-space data external to the central region of the k-space data array. The method acquires a motion signal indicating respiratory motion at least during the acquisition of the k-space data external to the central region and compares the motion signal with a predetermined threshold. The method identifies acquired k-space data corresponding to acquisition periods when the motion signal exceeds the threshold and excludes use of the identified acquired k-space data in image reconstruction using the remaining acquired k-space data.

LIU YILIN ET AL: "Four dimensional magnetic resonance imaging with retrospectivek-space reordering: A feasibility study", MEDICAL PHYSICS, AIP, MELVILLE, NY, US, vol. 42, no. 2, 1 February 2015 (2015-02-01), pages 534-541, presents the development and initial evaluation of a new strategy for 4D-MRI which is based on retrospective k-space reordering.

AKQAKAYA MEHMET ET AL: "Free-breathing phase contrast MRI with near 100% respiratory navigator efficiency using k-spacedependent respiratory gating : Improved NAV Efficiency for 4D Flow", MAGNETIC RESONANCE IN MEDICINE, vol. 71, no. 6, 30 July 2013 (2013-07-30), pages 2172-2179, investigates the efficacy of a respiratory motion scheme, where only the center of k-space is gated using respiratory navigators, versus a fully respiratory-gated acquisition for 3D flow imaging.

In diagnostic imaging, motion of the imaged patient is often a primary concern. Moreover, motion can even be considered to be one of the most important challenges for diagnostic imaging and in various treatments, such as radiation therapy (RT). Different sources of motion can be considered, some being more voluntary than others. Breathing motion is one type of motion that particularly needs to be considered, e.g. when scanning the torso, since it can easily lead to motion artefacts, which may severely impair the image quality of the acquired images, e.g. magnetic resonance (MR) images.

In magnetic resonance imaging (MRI), such as MRI scans acquired in the thoracic or abdominal region, it is important to suppress and/or account for the respiratory-induced motion. Luckily, for MR imaging there are different approaches known in the art to suppress this motion, e.g. breath-hold techniques and respiration-gated or respiration-triggered acquisition techniques.

In breath-hold approaches, many patients can have difficulties in suspending their respiration long enough for acquiring sufficient data (e.g. often more than 20 seconds), and, moreover, breath holds are difficult to perform consistently multiple times.

Gated and triggered acquisitions also have disadvantages. The configured gating time window can be set too small, which would lead to excessive scan times, while a window that is set too wide can lead to residual motion artefacts. The quality achieved by triggering, on the other hand, strongly depends on the accurate detection of the inhale or exhale and on the trigger delay, which may result, again, in motion artefacts.

Therefore, it is often not possible, or at least very difficult, for different imaging sequences in a single examination (e.g. spanning an extended time) to be acquired in exactly the same way, i.e. images will still be acquired in slightly different breathing states. For example, respiratory suppression techniques used in MRI all result in images showing a slightly different representation of the anatomy, since they are not acquired in exactly the same respiratory state, i.e. at a different amplitude and/or phase with respect to the respiratory cycle.

Image registration techniques can be used to reduce this discrepancy, e.g. to project the images into a common reference frame. For example, this allows the information from these MR images to be used effectively (and/or efficiently) in radiotherapy, e.g. to delineate the target to be treated and/or organs at risk, to plan the therapy in general and/or to assess the therapy result. For example, the images may be generally registered to each other, e.g. in which one scan is used as the master (reference image) to which other scans are registered (translated, reoriented, warped, deformed and/or otherwise projected onto the reference). This registration step can be computationally very challenging, e.g. due to different contrasts. Additionally, the MR images are also often registered to a CT image, which increases difficulty even more, since contrasts between these two image modalities differ significantly, rendering it more difficult to register the images properly. These factors can all contribute to errors, which propagate throughout the entire treatment and therefore increase overall uncertainty and inaccuracy.

For therapies that use medical imaging for planning or guidance, such as MRI for radiotherapy or high-intensity focused ultrasound (HIFU), image registration can reduce the overall treatment quality, since every registration step adds uncertainty and/or inaccuracy propagating throughout the entire therapy workflow, e.g. potentially causing more healthy tissue to be irradiated than strictly necessary in radiotherapy. Moreover, it is often not only required to have images with good image quality and limited (respiratory) motion artefacts, but the respiration may also need to be characterized, such that this breathing pattern can be taken into account during planning, e.g. to achieve better conformal radiation plans. For example, in radiotherapy planning, margins (e.g. defining an internal target volume) are typically added to a gross target volume to take the uncertainty induced by respiration into account. Therefore, better knowledge of the breathing pattern can enable the radiotherapist to plan the therapy within a narrower margin.

Therefore, determining the respiratory state accurately for each acquisition can reduce uncertainties and inaccuracies. This "respiratory state" can be described by a combination of the amplitude and phase within the respiratory cycle. The respiratory cycle is generally not symmetric, e.g. when comparing inhale and exhale, and can substantially vary across cycles, e.g. due to hysteresis within each respiratory cycle, changes in behavior, fatigue, and/or other influencing factors.

The respiratory state corresponding to each image (or raw image data) can be detected by continually tracking the relevant physiology and/or anatomical features, e.g. using a respiratory surrogate signal. Suitable signals can be determined by processing a camera observation video stream, using a breathing belt sensor, and/or by various other sensor technologies known in the art. It is also known to derive a (semi-)quantitative surrogate signal indicative of respiration directly from an output of the imaging system, e.g. an MR navigator signal, a noise navigator signal or another surrogate derived from the acquired data itself.

It is known in the art that the registration step between MRI and CT can introduce a systematic registration error of, for example, in a range of 2 to 5 mm, which will propagate throughout the entire radiotherapy planning and treatment. This would be a good impetus to rely on substantially MRI-only treatment planning, e.g. in which only MRI data is used to delineate the relevant anatomical features and plan the radiotherapy treatment. However, the registration between different acquisitions within a single MRI exam will also introduce a registration error.

For MRI-guided treatments, such as MR-guided radiotherapy, e.g. delivered by a combination of MRI system and radiotherapy accelerator (MR-Linac), it is particularly important that the patient is scanned in the same way during treatment as during simulation. By minimizing differences in positioning by various means, as commonly applied, the image registration can be performed relatively easy and accurate, e.g. such that identified contours can be quickly and accurately projected when generating a new radiotherapy plan. However, the respiratory state remains a constraining factor.

### SUMMARY OF THE INVENTION

It is an object of embodiments of the present invention to provide in good and/or efficient (e.g. automated; e.g. accurate) means and methods to associate a current respiratory state with magnetic resonance (MR) imaging acquisitions.

It is an advantage of embodiments of the present invention that image registration (e.g. of images within a single, e.g. prolonged and/or multi-sequence, MRI examination) can be avoided or can be performed accurately and/or efficiently.

It is an advantage of embodiments of the present invention that a good accuracy, efficiency and/or effectiveness can be achieved in radiotherapy, and/or for other image-guided therapies, e.g. HIFU.

It is an advantage of embodiments of the present invention that patient motion due to breathing can be accurately taken into account in MRI imaging.

It is an advantage of embodiments of the present invention that MRI imaging of the thoracic and/or abdominal region can be performed with good image quality, e.g. accurately, e.g. so that image artefacts due to motion can be reduced.

It is an advantage of embodiments of the present invention that good, e.g. short, scan times can be achieved in MRI imaging, e.g. as opposed to some prior arts methods, e.g. such as some prior art methods relying on respiration gating techniques.

It is an advantage of embodiments of the present invention that MRI images (and/or MRI raw imaging data) can be acquired with good knowledge of the associated breathing state of the imaged subject.

It is an advantage of embodiments of the present invention that multiple MRI images (and/or raw MRI data) can be acquired, e.g. in different sequences in the same examination session, in substantially the same breathing state (e.g. at the same breathing phase and/or amplitude, or an abstraction thereof), e.g. accurately at the same point in different breathing cycles.

It is an advantage of embodiments of the present invention that image registration of different MRI images, e.g. acquired within the same examination and/or in different MRI examinations, and/or also other image modalities, e.g. (an) additional CT image(s), can be accurately, efficiently and/or easier registered to each other (or to another reference image).

It is an advantage of embodiments of the present invention that image registration errors and/or artefacts in registered images can be reduced, and therefore that propagation of such errors/artefacts to further images, plans, treatments and/or other kinds of derived information can also be reduced if derived accordingly from and/or based on said registered images.

It is an advantage of embodiments of the present invention that image data can be acquired with good knowledge of the associated breathing state, such that this side-information can be taken into account in further use, e.g. in planning and/or assessing a therapy.

It is an advantage of embodiments of the present invention that uncertainty margins taken into account in planning a therapy, e.g. radiotherapy, can be reduced due to lower margins of error on the determined breath state associated with imaging data. Therefore, a good therapy can be achieved, e.g. potentially irradiating less healthy tissue and/or achieving a better dose delivery in the treatment target.

It is an advantage of embodiments of the present invention that a signal indicative of a breathing state can be determined and associated with corresponding acquired imaging data and/or images.

It is an advantage of embodiments of the present invention that registration errors (e.g. in terms of spatial mismatch) can be reduced and/or removed, e.g. systematic errors and/or random errors can be reduced.

It is an advantage of embodiments of the present invention that a high-quality MRI-guided treatment, e.g. MRI-guided radiotherapy, can be achieved. For example, differences in respiratory state during a pre-treatment imaging operation for simulation purposes and during treatment can be reduced. For example, by including accurate knowledge on the respiratory state for each acquired image (and/or the raw data it is based on) for a same patient, there is more certainty that differences between treatment and simulation are only due to underlying changes that should be considered when adapting the plan to take a treatment response and/or other anatomical/physiological changes into account.

It is an advantage of embodiments of the present invention that a motion model and a breath state signal (e.g. a "surrogate signal") can be combined to accurately identify the breathing state of each acquisition.

It is an advantage that the image acquisition can be automatically aligned to the (or each) breathing phase of interest, e.g. in reconstructing the k-space data (e.g. by adding a determined phase offset) and/or by taking this into account in image registration.

A method, computer-program product, and magnetic resonance imaging system as defined in claims 12, 13 and 1 achieves the above objective.

In a first aspect, the present invention relates to a method for respiratory monitoring of a subject's respiration during a magnetic resonance imaging session as defined in claim 12. The method comprises acquiring magnetic resonance imaging data comprising k-space values along at least one trajectory segment in a k-space matrix. The method also comprises monitoring the respiration of the subject by measuring one or more respiratory parameters. The method comprises prospectively determining a point in time when the respiration will correspond to a reference respiratory state, and control at least one acquisition parameter of an acquisition of the or each trajectory segment such that said point in time when the respiration will correspond to a reference respiratory state determines the time of sampling of the nearest point of the k-space trajectory segment to the centre of the k-space matrix by using the monitored at least one respiratory parameter, determining a point in time when the nearest point of the k-space trajectory segment to the centre of the k-space matrix was sampled in acquiring the magnetic resonance imaging data, determining the at least one respiratory parameter for said point in time using said monitoring of the respiration, and retrospectively comparing the determined at least one respiratory parameter corresponding to said time of sampling of the nearest point on the trajectory segment to the centre of the k-space matrix to a predetermined reference value corresponding to the reference respiratory state, to determine a phase offset from this comparison and to apply the phase offset to the k-space values obtained for the trajectory segment to compensate for a translation in real space due to respiratory motion as determined from said comparison.

A method in accordance with embodiments of the present invention may comprise providing feedback to the subject to guide the breathing behavior of the subject toward a predetermined breathing pattern. The predetermined breathing pattern may be fixed, e.g. immutable in the context of a specific use case of the method, may be configurable by a user (e.g. selectable or definable in terms of parameters by a technologist or medical worker), and/or may be determined by an automated method, e.g. by taking desirable properties of the images to be acquired and/or a medical situation of the subject into account.

A method in accordance with embodiments of the present invention may comprise determining the reference respiratory state by receiving reference information via a user interface and/or by retrieving the reference respiratory state from a data storage.

In a method in accordance with embodiments of the present invention, the monitored at least one respiratory parameter may be used to determine a plurality of points in time when the respiration will correspond to the reference respiratory state for respectively acquiring a plurality of trajectory segments such that each determined point in time determines the time of sampling the center of the k-space matrix and/or the nearest point on each k-space trajectory segment to the center of the k-space matrix.

In a method in accordance with embodiments of the present invention, controlling the at least one acquisition parameter for each of said plurality of trajectory segments may comprise taking a predetermined time window for the collective acquisition of the plurality of trajectory segments into account and adaptively applying a sparse sampling and/or compressed sensing technique toward and/or past the predetermined time window. For example, sparse sampling may be combined with an iterative minimization approach for reconstruction, or with another reconstruction technique that is suitable for sparsely sampled data acquisition.

A method in accordance with embodiments of the present invention may comprise registering previously acquired images of said subject, having respiratory information associated with said previously acquired images, modelling transformations obtained in said registration as function of the respiratory information to obtain a motion model, and determining said phase offset using said motion model.

A method in accordance with embodiments of the present invention may comprise reconstructing the acquired magnetic resonance imaging data and/or the magnetic resonance data corrected by said determined phase offset into at least one tomographic image, wherein said tomographic image is annotated with a breathing state that is determined by the at least one respiratory parameter determined to correspond with said point in time of sampling the center of k-space and/or said predetermined reference value used for determining said phase offset.

In a method in accordance with embodiments of the present invention, reconstructing said at least one tomographic image may comprise selecting a subset of the magnetic resonance imaging data such as to fill the k-space matrix with the selected magnetic resonance imaging data for reconstruction, in which acquisitions of k-space trajectory segments are selected based on a relation of the at least one respiratory parameter determined for each k-space trajectory segment with respect to a reference respiratory state to reconstruct.

In a method in accordance with embodiments of the present invention, acquiring the magnetic resonance imaging data may comprise repeatedly acquiring a same k-space trajectory segment at different points in the respiratory cycle, such that images can be (and/or are) reconstructed for different respiratory states by selection from, interpolation between and/or weighting of different sets of data acquired for said same k-space trajectory segment.

In a second aspect, the present invention relates to a computer-program product as defined in claim 13.

In a third aspect, the present invention relates to a magnetic resonance imaging system as defined in claim 1, the system comprising a device for respiratory monitoring of a subject's respiration during a magnetic resonance imaging session. The device comprises a sensor input for receiving sensor data and determining at least one respiratory parameter therefrom to continually monitor the respiration of the subject during the magnetic resonance imaging session. The device comprises a data input for receiving magnetic resonance imaging data comprising k-space values along at least one trajectory segment in a k-space matrix, and a processor. The processor is configured to perform the inventive method as defined in claim 12.

In a system in accordance with embodiments of the present invention, the controller output may be adapted to provide a trigger signal to the magnetic resonance imaging system on which the magnetic resonance imaging session is performed to initiate the acquisition of the magnetic resonance imaging data for said k-space trajectory segment at a time determined such that the time of acquiring the center of k-space data, or the closest point on the trajectory segment thereto, coincides with said point in time when the at least one respiratory parameter is predicted to correspond to said reference respiratory state.

In a system in accordance with embodiments of the present invention, the processor may be adapted to use the monitored at least one respiratory parameter to determine a plurality of points in time when the respiration will correspond to the reference respiratory state for respectively acquiring a plurality of trajectory segments such that each determined point in time determines the time of sampling of the center of the k-space matrix and/or the nearest point on each k-space trajectory segment to the center of the k-space matrix.

In a system in accordance with embodiments of the present invention, the processor may be adapted to control the at least one acquisition parameter for each of said plurality of trajectory segments taking a predetermined time window for the collective acquisition of the plurality of trajectory segments into account, and to adaptively apply a sparse sampling (e.g. combined with a suitable reconstruction technique) toward and/or past the predetermined time window.

In a system in accordance with the present invention, the processor is adapted to retrospectively compare the determined at least one respiratory parameter corresponding to said time of sampling of the nearest point on the trajectory segment to the center of, the k-space matrix to a predetermined reference value corresponding to the reference respiratory state, to determine a phase offset from this comparison and to apply the phase offset to the k-space values obtained for the trajectory segment to compensate for a translation in real space due to respiratory motion as determined from said comparison.

A system in accordance with embodiments of the present invention may comprise a data storage, wherein said processor may be adapted to retrieve previously acquired images of said subject from the data storage, in which respiratory information is associated with each of said previously acquired images. The processor may furthermore be adapted to register the previously acquired images, to model transformations obtained by said registration as function of the respiratory information to obtain a motion model and to determine said phase offset to compensate for a translation in real space due to respiratory motion using said motion model. Alternatively, the system may be adapted to receive such registration and/or such motion model directly from the data storage, e.g. where it may be stored by an external image processor.

A system in accordance with embodiments of the present invention may comprise a reconstructor for reconstructing the received magnetic resonance imaging data and/or the phase-corrected magnetic resonance imaging data into at least one tomographic image.

In a system in accordance with embodiments of the present invention, the reconstructor may be adapted to annotate the reconstructed tomographic image with a respiratory state that is determined by the at least one respiratory parameter that is determined to correspond with said point in time of sampling the center of k-space and/or by said predetermined reference value used for determining the phase offset to adjust the magnetic resonance imaging data used for the reconstruction.

In a system in accordance with embodiments of the present invention, the reconstructor may be adapted to select a (proper) subset of the magnetic resonance imaging data such as to fill the k-space matrix with the selected magnetic resonance imaging data for reconstruction, in which k-space trajectory segments are selected based on a relation of the at least one respiratory parameter determined for each k-space trajectory segment with respect to a reference respiratory state to reconstruct.

In a system in accordance with embodiments of the present invention, the processor may be adapted to control the acquisition of the magnetic resonance imaging data by repeatedly acquiring a same k-space trajectory segment at different points in the respiratory cycle in an oversampling strategy. The reconstructor may be adapted to reconstruct images for different respiratory states by a corresponding selection from, interpolation between and/or weighting of data acquired for said same k-space trajectory segment.

A system in accordance with embodiments of the present invention may comprise a feedback output for providing sensory feedback to the subject during the magnetic resonance imaging session to guide the breathing behavior of the subject toward a predetermined breathing pattern.

A system in accordance with embodiments of the present invention may be configured such the sensor data is synchronized to the magnetic resonance imaging data.

A system in accordance with embodiments of the present invention may have the processor configured to determine the point in time when the nearest point of the or each k-space trajectory segment to the center of the k-space matrix is sampled at least partially using the at least one acquisition parameter. The processor may for example have access to pulse sequecne commands which define the timing of various gradient and radio frequency pulses as well as acquisition times for the magnetic resonance data. This may enable determinination of the point in time when the nearest point of the or each k-space trajecctory segment to the center of the k-space matrix is sampled at least partially using the at least one acquisition parameter.

The independent and dependent claims describe specific and preferred features of the invention. Features of the dependent claims can be combined with features of the independent claims and with features of other dependent claims as deemed appropriate, and not necessarily only as explicitly stated in the claims.

### BRIEF DESCRIPTION OF THE DRAWINGS

Fig 1 shows a method in accordance with embodiments of the present invention.
Fig 2 shows a device in accordance with embodiments of the present invention.
Fig 3 illustrates a magnetic resonance imaging system in accordance with embodiments of the present invention.

The drawings are schematic and not limiting. Elements in the drawings are not necessarily represented on scale. The present invention is not necessarily limited to the specific embodiments of the present invention as shown in the drawings.

### DETAILED DESCRIPTION OF EMBODIMENTS

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

In a first aspect, the present invention relates to a method for respiratory monitoring of a subject's breathing during a magnetic resonance imaging session, e.g. to annotate imaging data and/or to take a subject's breathing into account during the procedure and/or in postprocessing of the acquired data.

It will be understood that the magnetic resonance imaging (MRI) session refers to an examination using an MR imaging scanner, e.g. for diagnostic and/or medical purposes, such as to diagnose a condition of the subject, to plan a treatment, to monitor and/or control a treatment that is performed concomitantly, and/or other related, e.g. medical purposes. The examination may comprise the magnetic field gradient and radiofrequency pulse application and data acquisition for one or more MRI sequences, e.g. to reconstruct one or more images, e.g. tomographic and/or 3D images with possibly different contrast characteristics and/or to show dynamic changes in the body, e.g. one or more 4D scans. An example of a use case in which a therapy is concomitantly performed is an (MR) image-guided radiotherapy, e.g. using a combination of a magnetic resonance imaging system and a radiotherapy accelerator (MR-Linac). Where reference is made to a session, it will be understood that this may refer to a procedure in which the subject is positioned in a magnetic resonance imaging system for performing the imaging procedure, after which a plurality of different sequences may be applied to acquire MRI data, e.g. without removing the patient from the imaging environment (not necessarily excluding potentially repositioning the patient in a different pose or to better align the body). Cases in which the subject is removed from the imaging system to return for a further session at a later time or date may generally be considered a plurality of sessions, notwithstanding that embodiments of the present invention can also be advantageous for improving consistency and/or improving image quality when applied equally in multiple sessions of such multisession series.

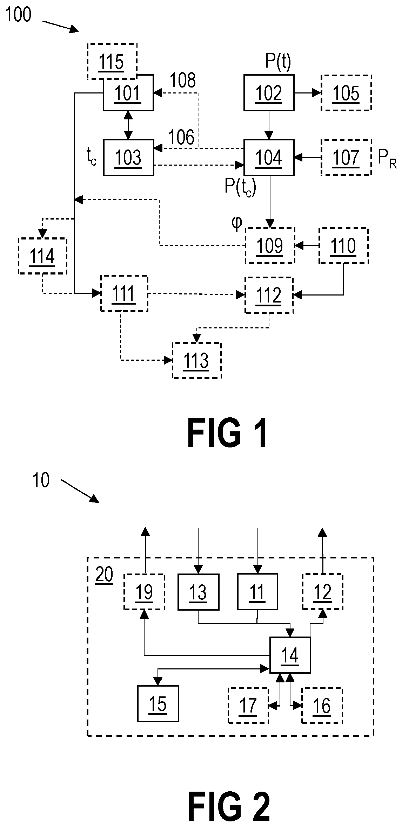

Referring to Fig 1, an illustrative method 100 in accordance with embodiments of the present invention is shown. The method comprises monitoring 102 the respiration of the subject by (e.g. continuously and/or continually, e.g. at least frequently) measuring one or more respiratory parameters *P(t),* e.g. using a sensor. Thus, the respiratory parameter(s) may be continually sampled in a consistent manner.

Generally, the sensor may refer to any suitable sensing device, i.e. a device or system capable of determining a relevant parameter that can characterize an aspect of the respiration from an observed physical quantity or quantities. The respiratory parameter may be a parameter that is clearly directly related to respiration, e.g. air volume in the lungs, in/exhaled air flow and/or breathing frequency, and/or that is sufficiently related to and/or indicative of (a) characteristic(s) of the respiration cycle (e.g. a "surrogate" signal). A combination of different parameters may be used to more fully characterize the respiration, e.g. a phase value within the respiratory cycle and an amplitude value of the breathing (e.g. indicative of momentary chest expansion). Examples of suitable signals that can be considered as a respiratory parameter and/or from which such a parameter can be derived include (a) monitored predetermined reference position(s) (or changes thereof) on the chest due to breathing, a velocity or acceleration measurement of such reference position(s), measurements of expansion of the chest, pressure measurements and/or relevant information derived from camera observation images (e.g. a video stream) by image processing techniques. This also implies that the sensor or sensing device can take many forms, e.g. may comprise a spirometer, a proximity and/or ranging sensor, a breathing belt sensor, an accelerometer, a radar sensor or similar technology (lidar, sonar, ...), a pressure sensor or array of pressure sensors (e.g. arranged in a pressure-sensing mattress) and/or a camera or combination of cameras (e.g. arranged for depth imaging), without limitation to these examples. When the sensor or sensing device comprises one or more cameras, this/these can comprise a monochrome camera, a color image camera, and/or a multispectral camera, and/or can operate in the (human) visible spectrum, the infrared spectrum, a combination thereof, or a small sub-band thereof (e.g. only a specific color component). The at least one respiratory parameter may be derived from the data provided by the sensor or sensing device in a straightforward manner (e.g. the sensor may provide an output that is directly usable as a respiratory parameter of interest) or may require some processing, e.g. to determine a robust parameter (e.g. correlated with or at least indicative of a current air volume in the lungs, a chest expansion level, a breathing frequency, ...) from the data, e.g. from a video stream. However, many suitable methods are known in the art to determine one or more respiratory parameters from various data sources (including, for example, video camera observation), and can thus be readily implemented in embodiments in accordance with the present invention.

The method may thus comprise acquiring sensor data from one or more sensors and/or one or more sensing devices (which may also include, for example, camera observation systems from which relevant information can be derived), and determining the one or more respiratory parameters from the acquired sensor data, e.g. by applying suitable processing, e.g. to determine one or more parameters indicative of breathing, e.g. a measure (e.g. point-measure in time) indicative of a current air volume filling the lungs and/or another generalized indicator of breathing, e.g. a breathing velocity, a breathing rate, a distance between reference points where at least one point is subject to motion due to breathing, and/or other such information indicative of respiration.

MRI data may be acquired during free-breathing, or may be combined with other motion suppression techniques known in the art. For example, the monitoring of the respiration may be used to provide 105 feedback to the patient to coach the breathing behavior toward a predetermined breathing pattern, as known in the art. Feedback may thus be provided to the subject being imaged to influence the subject to breathe in accordance with the predetermined breathing pattern. This allows the acquired data to be better aligned with (a) predetermined breathing state(s), and may improve the robustness and reproducibility of the imaged breathing state(s). For example, breathing information may be shown or otherwise conveyed to the patient, e.g. in combination with information on a desired breathing pattern, e.g. by visualizing deviations therefrom or providing other sensory cues to represent such deviations. This may be applied during the imaging procedure, e.g. simulation imaging for a therapy, and may later be repeated during a treatment, such that the breathing pattern corresponds, i.e. such that the gathered imaging data forms an accurate representation of the conditions during treatment.

Thus, the method may comprise providing 105 feedback to the subject to influence the subject's breathing while monitoring the respiration in order to meet or approximate a predetermined or selected breathing goal. Thus, the subject can be actively guided toward a breathing pattern that corresponds to the goal. For example, the measured respiratory parameter(s) may be compared to a target defined by the goal and a feedback may be provided to the subject that represents the currently observed parameter(s) contrasted with the target, a difference or other type of deviation measure between the current and target values, or in another way to enable the subject to gain insight in the alignment of his or her present breathing pattern with the predefined or selected goal.

Such coaching can take various forms and shapes, potentially based on multi-modality input, e.g. different types of sensor input and/or different measured or derived parameters representative of aspects of breathing. A typical example is displaying the desired breathing pattern and providing insight or feedback on the accuracy of the actual breathing pattern. For the latter, the method may check in how far the actual behavior is near to the desired goal behavior to provide suitable feedback.

As an example, WO 2020/069948 A1 discloses a suitable method for influencing a breathing parameter of the subject's breathing pattern in order to meet the goal. In this illustrative approach, the breathing parameter can be decreased or increased (toward a target in line with the goal) by monitoring a current value of the breathing parameter and providing sensory feedback to the subject about a determined preferred value (different from the current value) of the breathing parameter for achieving the goal. For example, the preferred value of the breathing parameter may be determined by means of an artificial intelligence intelligent agent, as described in the referenced publication. However, other approaches for coaching the breathing pattern toward a specific goal are also known in the art and not necessarily excluded.

The method 100 comprises acquiring 101 magnetic resonance imaging data comprising k-space values along a trajectory, e.g. a line, in k-space in one or more acquisitions, e.g. to sample and fill a k-space matrix or matrices to reconstruct one or more images from, e.g. by filling a plurality of lines and/or trajectory segments through the matrix until sufficient (or all) values in the matrix are sampled to allow MRI image reconstruction. As well-known in the art, this may comprise applying a strong magnetic field around the body region to be imaged, e.g. by positioning the patient in a magnet bore, e.g. of a strong superconducting magnet, in which a strong magnetic field is present, e.g. in the order of at least 0.1T, e.g. at least 0.5T, typically 1T to 4T, or even higher, e.g. 4T to 10T. This main magnetic field generally aligns, e.g. polarizes, magnetic moments of susceptible atoms in the body, e.g. hydrogen atoms.

Various magnetic field gradients may be applied to cause differences in the magnetic field across the body, e.g. to select different planes, generally regions, in the body to image and/or to encode spatial information in a signal response by various possible encoding mechanisms, e.g. phase and/or frequency encoding. A radiofrequency pulse, e.g. typically as part of a series of pulses or other transmitted RF signals, may be applied such that an oscillating magnetic field is temporarily obtained at an appropriate resonance frequency, e.g. by the Larmor precession. For example, gradient coils may condition a selected region of the patient's body to the required magnetic field conditions to absorb the energy of the pulse and resonate, i.e. such that a radio frequency (RF) signal is emitted in response, which may then be measured by a receiving coil. Processing of the response RF signal allows information to be deduced about the material at its resonation origin. By further varying the applied gradient field(s) and repeatedly applying and acquiring RF signals, information can be reconstructed about local composition and/or structure variations in the body, e.g. to reconstruct one or more tomographic images, e.g. 2D images, 3D images and/or 4D images (i.e. a temporal sequence of 3D images) of the internal structure of the body.

By combining this with the information from monitoring 102 the respiration with the MRI acquisition 101, the respiratory state can be characterized at the time of data acquisition. Thus, images can be reconstructed from data that are acquired in the same respiratory phase. Therefore, the need for registration steps can be reduced or eliminated, or can be more easy to perform (e.g. by reducing the initial difference between images to be registered). By monitoring the respiration using a suitable respiratory (e.g. surrogate) signal, e.g. preferably a signal that does not automatically rescale and is captured at a sufficiently high spatial resolution (or can be interpolated or modeled accurately based on less frequent sampling), an accurate characterization of the breathing can be obtained, which, in combination with accurate knowledge of when MRI data is being acquired, allows the respiratory state of each acquisition to be defined.

The moment in time at which the center of k-space is sampled for the data from which an image is reconstructed can be used as a good representation for the time at which the image is acquired as a whole, such that the respiratory state associated with an (e.g. 2D and/or 3D) image can be determined by correlating the instant in time of center k-space sampling with the respiratory surrogate signal.

Generally speaking, in MRI, k-space refers to the (complex-valued) two- or three-dimensional Fourier space of the MR image (volumetric image or 2D image slice) being acquired. The k-space of an (planar or volumetric) image is being sampled by applying a carefully controlled pulse sequence, e.g. an accurately timed sequence of radiofrequency pulses and field gradients, to obtain a matrix of k-values, which forms a temporary image space. Typically, the k-space matrix is filled progressively (therefore, spread in time) along a sampling trajectory, e.g. row by row (or Cartesian), in a radial sampling pattern, in a zig-zag pattern, in a spiral pattern, and/or possibly less common patterns. Each line or trajectory segment being sampled through the k-space matrix has a point where it passes as near as possible to the center of k-space, where the lowest spatial frequency for that line or segment is encoded.

The time at which data for the center of the k-space matrix is acquired is accurately known a priori (and a posteriori), e.g. in relation to the applied sequence. The temporary image of k-space values can then be processed, e.g. reconstructed, to obtain the desired image (or part thereof). For example, the k-space matrix may have the same number of rows and columns as the final image to be reconstructed, and is filled line per line (or using another trajectory) with raw data during the scan, e.g. one line per repetition time TR (without necessarily limitation thereto).

The method 100 comprises, determining 103 the point in time *t_{c}* when the center (e.g. origin) of the k-space matrix and/or the nearest point of the k-space line (e.g. a row) or trajectory segment to the center of k-space is sampled in acquiring 101 the magnetic resonance imaging data, e.g. retrospectively for an acquisition that was already performed and/or prospectively for an acquisition to be executed (e.g. by adjusting acquisition parameters and/or triggering).

It will be understood that the matrix is not necessarily comprised of an odd number of rows and/or columns, nor an individual line or segment an odd number of pixels, such that the center (and/or the point of nearest passing) may refer to an interpolated point between the center or closest pixels (e.g. averaging the points in time at which four pixels of the center 2x2 block are sampled). Alternatively, one of the pixels that are closest/nearest (and mutually equally close/near) to the center may be selected as reference. Regardless of the matrix having an even or odd number of rows/columns, a small neighborhood of the center may be used for averaging (or otherwise aggregating) the sampling times to obtain the representative point in time where the center is sampled for increased robustness. The neighborhood may be symmetric, e.g. a block, an approximation of a disk, or another suitable form, but may also be asymmetric, e.g. considering a plurality of center pixels of a single center row. Preferably, if such averaging (or otherwise aggregating) is used, the number of pixels (k-values) considered may be rather small, e.g. less than 20 pixels, preferably less than 10 pixels, e.g. 9, 4 or 2. It is also to be noted that the point in time of sampling the center of k-space (or the nearest pass of a trajectory segment) may be determined from the sequence directly, and may often correspond to the middle of the acquisition time window for a single acquisition (but not necessarily so).

The time of sampling the center of k-space may be determined for each line in the k-space matrix, e.g. corresponding to only the center in one k-space dimension (e.g. center column of any row), or only for a central line (e.g. center column of center row). In other words, the center of k-space may refer to the center in one dimension, in two dimensions or in three dimensions, as determined for each line, respectively over all lines. Embodiments are not necessarily limited to row by row sampling, i.e. the k-space line may generally refer to any trajectory (or part thereof) through k-space that is being sampled and the point in time that is determined 103 may correspond to the time at which the data acquisition sequence crosses or comes nearest to the center of k-space in at least one k-space dimension, e.g. where the lowest spatial frequency component for that trajectory (segment) is captured.

The monitored 102 at least one respiratory parameter *P(t_{c})* is also determined 104 for this point in time *t_{c},* e.g. by predicting the respiratory parameter at the time of interest based on previous measurements of the parameter (i.e. when determining *P(t_{c})* prospectively) and/or by looking up, interpolating and/or otherwise relying observed values of the respiratory parameter(s) (when retrospectively determined). Therefore, the respiratory state that is representative for each acquisition (each k-space line or trajectory segment, and/or each k-space matrix as a whole) is determined, which can for example then be used to combine all acquisitions for the same respiratory phase, e.g. in reconstruction, and/or to annotate reconstructed images with the corresponding respiratory state.

Since values near the center of k-space correspond to low-frequency (spatial) Fourier components, it will be understood that these values will have the strongest effect on the reconstructed image, e.g. affecting large-scale features of the reconstructed image. Therefore, even though a data acquisition for an image is generally spread in time, the time of acquisition of these low-frequency components forms the best representation of the time of acquisition for the image as a whole, and likewise, the crossing or nearest passing to the center of k-space for each individual acquisition that samples a single line or trajectory segment in k-space forms the best representation for the time of acquisition of that line of segment. The breathing state as determined by the monitored respiration at this time is thus also a good approximation of the breathing state associated with the image as a whole, or, likewise, with the specific trajectory segment.

In a method in accordance with embodiments of the present invention, the monitored 102 at least one respiratory parameter *P(t)* is used 106, prospectively, to determine the point in time *t_{c}* where the center of k-space and/or the point nearest to the center for a k-space line or trajectory (or trajectory segment) will be sampled, e.g. using a predictive model of the respiratory parameter(s) to predict the time *t_{c}* at which the respiratory parameter(s) *P(t_{c})* is expected to be in a predetermined state, e.g. such that *P(t_{c}) = P_{R},* where *P_{R}* represents the value(s) of the respiratory parameter(s) corresponding to that predetermined state. This may be as simple as applying a fixed time offset (between the start of an acquisition and reaching the most central k-space sampling position for that acquisition), or may use a more elaborate model of the acquisition process and/or the time evolution of the respiratory parameter(s). The MRI acquisition may be triggered accordingly such that the target respiratory state coincides with the time of sampling the center of k-space, e.g. taking a predetermined delay into account between the trigger and the point in time when the center of k-space will be sampled. Additionally or alternatively, e.g. instead of or in addition to controlling a time at which an acquisition is started (e.g. by generating a trigger), properties of the applied field gradients may be varied to shift the time of acquisition of the center of k-space to the target as determined from the respiratory monitoring.

This approach allows the acquisition of the (most) central k-space data to correspond to the body being in the predetermined respiratory state. Optionally, actual measurements obtained after the acquisition can be used post facto to define the respiratory state even more accurately, e.g. to adjust the respiratory parameter(s) associated with the acquisition to the actual respiratory state at the time of center of k-space acquisition if needed, e.g. replacing the prospective target value with a value based on actual measurements 102.

In other words, the monitored 102 at least one respiratory parameter is used to determine 104 a point in time where the respiration will correspond to (at least approximatively) a reference respiratory state, and this point in time is used 108 to determine and apply acquisition parameters (e.g. a time for triggering the acquisition and/or other acquisition parameters) for acquiring 101 the MRI data, such that the determined 103 point in time of sampling the center of the k-space matrix and/or the nearest point on the k-space line or trajectory segment to the k-space center in accordance with the determined acquisition parameters coincides with the point in time where the respiration will be in the reference respiratory state.

Therefore, the point in time of acquiring the center of k-space of a raw k-space image (and/or the most central point of each trajectory segment being sampled for the image) is timed to the co-occurrence of a predetermined state of the respiration. The reference respiratory state may be determined as a phase of the breathing cycle (e.g. a time point with respect to a reference starting point of the cycle) and/or other parameter(s), e.g. a respiratory amplitude, that is/are clinically of interest, or may be defined by a previously acquired image, e.g. in a different imaging session. For example, the breathing parameter(s) (or abstraction thereof) associated with an earlier acquired image may be used as reference for a new acquisition.

The method may comprise determining 107 the reference respiratory state (e.g. the corresponding reference parameter value or values *P_{R}*, or an abstraction thereof) by interaction with a user, e.g. receiving the reference respiratory state or information to identify that state from the user via a user interface, e.g. a graphical user interface, and/or by retrieving the reference respiratory state from a data storage, e.g. as an annotation added to a previously acquired image and/or stored in association with such previously acquired image.

An example of the latter approach may relate to applying the method 100 in a follow-up examination, in which the evolution of a medical condition with respect to an earlier situation is of interest, for example to evaluate the response to a therapy. This allows the new acquisition to be tuned so as to acquire an image or images that are representative of the same anatomical/physiological situation as the respiratory state visualized by the previously acquired image(s). Another example may be a radiotherapy workflow, in which a first MRI or CT image, appropriately annotated with respiratory information, may be used as reference to align the breathing state of a new MRI image to be acquired to, such that new information can be gathered for the same respiratory state, e.g. adding MRI information to previously collected CT and/or MRI data and/or to align MRI imaging during a therapy session (e.g. using an MR-Linac combination) with a planning master image.

For example, in the same imaging session or in a previous imaging session, respiratory information and associated imaging data may already have been acquired, and this information may be used to determine a reference respiratory state of interest, such as the maximum exhale (e.g. typically most stable for planning a therapy) or a mid-position (e.g. the point where a volume of interest is in its position where it is found statistically on average over a breath cycle, which may be most representative for planning a therapy). The target reference respiratory state of interest may also be determined directly, e.g. using a predictive model, and/or from the monitored respiratory surrogate signal, and/or simply by selection by an operator, e.g. via a user interface.

Therefore, the acquisition of k-space MRI data may be prioritized for a specific point in the breath cycle, i.e. the reference state. The data may be acquired in such a way that the center of k-space is always acquired in the desired respiratory phase, while the data close to the center of k-space is acquired in the time just before or just after the most optimal respiratory phase. Peripheral k-space data may thus be acquired in a different respiratory phase, e.g. depending on the required time for a line acquisition. However, these peripheral data may typically have a less profound impact on the reconstructed image quality, since they relate to progressively smaller spatial scales relative to the center of k-space, while also correlating with increasing time differences with respect to the target point in the breath cycle as the distance in k-space increases.

For example, the method may be configured to take a target or maximum scan time into account, and select appropriate points in time *t_{c1},t_{c2},...* within this time window to optimize the data acquisition, e.g. to show minimum artefacts due to both motion and under-sampling. In other words, for each appropriate time point *t_{ci}* found (i.e. predicted) where the reference respiratory state will be achieved, one or more k-space lines (or generally trajectory segments) may be scheduled to coincide the collection of the center of k-space data (or nearest pass for that line) with the target point in time. Thus, an image may be collected by sampling parts of the k-space during different breath cycles, each time at or near the same target point of the respiratory cycle.

If it is determined that insufficient data is acquired near the end of the available time window, compressed sensing algorithms may be applied to help reduce under-sampling artifacts, e.g. using Poisson disk distribution sparse sampling. Thus, the method 100 may comprise using 106 the at least one respiratory parameter to determine a plurality of points in time for passing the center of k-space, or nearest thereto along its line or trajectory segment, for a plurality of acquisitions 101 tracing corresponding lines or trajectory segments through k-space, such that said points in time correspond to the target reference respiratory state. This use 106 may comprise optimizing 108 (e.g. determining, adapting) acquisition parameters such as to sample the k-space matrix sufficiently within a predetermined time window, e.g. fitting as much of such acquisitions into the time window as possible that at least approximate the intended target reference respiratory state and potentially reducing the time required for acquisitions by sparse sampling, e.g. toward or past the end of the fixed/preferred time window.

Therefore, the method may continually optimize 108 (e.g. update and/or change when needed) the sequence characteristics to apply, e.g. not necessarily only by triggering but potentially also other parameters, such as gradients, determine the delay in time of sampling the center of k-space (or nearest passing for a trajectory segment) for the next sequence part to apply with respect to initiation, determine the time at which the next sequence part needs to start to align the center k-space sampling with the desired respiratory state, and trigger the initiation of that acquisition accordingly.

The method in accordance with the invention comprises, after (e.g. after each, or after all) acquisition(s) 101, comparing the determined 104 at least one respiratory parameter *P(t_{c})* corresponding to the center of (or nearest point to center of) k-space of an acquisition to a predetermined reference value *P_{R},* e.g. a desired respiratory state to be examined or a respiratory state in which a previously acquired image of the same patient was acquired (cf. examples hereinabove), determining 109 a phase offset *φ(P(t_{c})-P_{R})* from this comparison, e.g. commensurate with a difference or other comparative measure (e.g. ratio) between the determined value and the reference, and adding (or subtracting, depending on the selected convention of sign in calculating the phase offset) the phase offset to the k-space values (e.g. of the line or trajectory segment) obtained in that acquisition. Thus, retrospectively, an additional phase is added during (or at least before) reconstruction to each acquired k-space line or set of lines (or each trajectory, each sub-part of a trajectory, the entire k-space matrix, ...). In an example outside the scope of the present invention, this step is not necessarily combined with the prospective tuning, e.g. by triggering, of the acquisitions to a desired reference respiratory state, as discussed hereinabove, nor is the reference value *P_{R}* for this corrective step necessarily the same as a reference value *P_{R}* when used for such prospective acquisition control. However, it will be understood that such combination, in accordance with the invention, advantageously enables an efficient correction of (e.g. typically small) deviations from the target.

Since different respiratory states will cause shifts in the underlying anatomy, a large component of the breathing induced differences can be compensated by a translation, which corresponds to a phase shift in k-space. A suitable correspondence between the difference of the monitored respiratory parameter with respect to its reference and the phase offset to be applied can be easily determined by experimentation and/or simulations. This correspondence may take additional information into account, e.g. such as to take an orientation and/or position of a k-space line in k-space into account, as well as potentially other parameters, such as patient characteristics (e.g. weight, volume, chest circumference, ...), a pose of the patient, a type of examination, and/or other such parameters.

It is an advantage that compensating for (e.g. residual) movement with respect to a reference breathing state can thus be performed easily in Fourier space. Even though this only compensates for a translation, it is to be noted that this approach can be applied to each line or set of lines acquired in k-space, and the dependence of the phase offset to be applied on the determined difference in breathing state can be tuned for each line or set separately (e.g. taking line orientations and/or positions into account). The result may thus be closer to a non-rigid transformation than to a mere translation in real image space (not necessarily excluding simpler embodiments where a same phase/respiratory parameter difference relation is used for all lines forming a raw image).

As an example, embodiments not being limited thereto, k-space may be sampled by a plurality of radial lines, each passing through the center, and a phase offset for each line may be determined as function of the radial angle and the observed difference between the respiratory parameter and its target. This would allow a simple compensation of the breathing motion by taking the distribution of chest volume expansion/contraction during breathing into account, e.g. at least for small differences between the target state and the determined respiratory state.

For example, the phase shift determined from the detected deviation between (the) observed breathing parameter(s) at the point in time of sampling the center of k-space with respect to a predetermined reference (e.g. target) for that/those breathing parameter(s) can be applied to the data before performing a Fourier transform (or similar reconstruction algorithm) to correct the data to (or toward) the correct respiratory phase.

In an illustrative approach, the required phase offset, e.g. the relation between phase offset and the respiratory parameter(s), can be determined easily by continuously (or continually, e.g. at a sufficiently high temporal resolution) acquiring a single k-space line or set of lines over a single (or averaged over a few) respiratory cycle, e.g. to form a calibration. At least in so far that the gradients are configured the same while acquiring this same line repeatedly, any changes in the observed signal over the breath cycle can be attributed to anatomical motion. Thus, the observed differences in phase over time in the k-space data are a result of (in first approximation) the translation of the underlying anatomy. Such calibration can be performed for a specific patient or specific session, or may be performed for a test population to determine a relation that is suitable for similar patients. This can easily be extended to incorporate variable parameters, such as patient weight, chest circumference, gender and/or other patient properties and/or to take a position and/or angulation of the k-space line and/or other sequence parameters into account, e.g. to determine a suitable relation that is dependent on such patient and/or sequence parameters.

It is also to be noted that, e.g. if an oversampling and/or free-breathing strategy is used, in a first stage, points in the respiratory cycle may be selected for which sufficient k-space lines are available (e.g. sampling of the k-space center or nearest passing thereof sufficiently close in time to the desired point in the respiratory curve). Images for these respiratory reference points may thus be reconstructed and used to construct a motion model, e.g. by registration 110 of the reconstructed images to each other (e.g. to any reference) and/or modelling the resulting transformations (e.g. deformable vector fields) as function of the respiratory parameter(s), e.g. respiratory phase. This allows the phase offset to be determined 109 for any k-space trajectory to project one point in time on the respiratory point to another, arbitrary point, e.g. using such motion model. This empiric relationship can be used to adjust k-space lines to any arbitrary respiratory state, such that in a second stage, phase-corrected data can be used to refine the first reconstructions and/or to reconstruct intermediate phases for which initially not sufficient data was available. In other words, the calibration can be carried out inline without requiring acquisitions specifically aimed at calibration only, in so far sufficient data is sampled over the entire breathing cycle to reconstruct a limited number of images sufficiently distributed over the respiratory cycle.

The method 100 may thus comprise determining a motion model from a registration 110 of previously acquired images with respiratory state information associated therewith, e.g. included as metadata in the images, and determining the phase offset *φ(P(t_{c})-P_{R})* to apply to the k-space line or segment from the motion model. The previously acquired images may be images of the same subject acquired in the same or a different imaging session, possibly even a different modality (e.g. CT). For example, a plurality of images (of the same patient showing the same, or at least sufficiently overlapping, anatomy) may have been acquired previously, e.g. as a 4D scan, such as a timeseries of 3D images spanning a breath cycle. These images may be registered to each other using image registration techniques known in the art, e.g. such that the different breathing phases in the 4D set are registered to each other (e.g. to one thereof selected as reference, without limitation thereto) in order to define a transform from one (resp. each) respiratory state to (e.g. any) other respiratory state. Such transform may for example be defined as a deformable vector field, or as another suitable rigid or non-rigid transformation.

Since a breathing state for the acquired data is accurately defined in accordance with embodiments of the present invention, the transformation(s), e.g. deformable vector field, determined from the previously collected data can be used to transform the (presently acquired) MRI data from the known respiratory state to any other desired state, e.g. corresponding to one of the previously collected and registered images or even by interpolation between those states. Even if the MRI data is not collected in exactly the same respiratory state as one of the previously acquired images, interpolation techniques and/or a motion model fitted to that data, e.g. based on the deformable vector fields, may be used.

It is an advantage of the phase adjustment technique 109 discussed hereinabove that, as opposed to a conventional (real-space) registration, the raw data can be composed of phase-adjusted data from different respiratory phases, e.g. even if not sufficient data is available for a reconstruction for any of the respiratory phases as such.

However, it will be understood that a conventional (real-space) registration can be used as well, additionally or alternatively, e.g. using a motion model (e.g. or directly a transformation) determined from registration of previously gathered images. Thus, the MRI data may be reconstructed 111 into an image with the breathing state corresponding to the time of sampling the center of k-space associated with the reconstructed image, and the reconstructed image may be transformed 112 by a suitable transformation determined from the previously registered images, e.g. using a parametric model, interpolation of vector fields or another suitable technique, to obtain a new image in any desired breathing state that is different from the state associated with the original reconstructed image. Since the previously acquired image data, e.g. 4D image set (e.g. a 4D synthetic CT image constructed from MRI data, without limitation to this example), may have been collected under conditions that are consistent (intra-series), e.g. each 3D image (time point) having the same resolution, field of view and/or substantially the same signal-to-noise ratio and contrast properties, the registration performed on those images may be robust and/or relatively easy. The information from this registration, e.g. in the form of a motion model, may be leveraged to transform the newly acquired data, e.g. even if the acquisition conditions are substantially different, e.g. on a different imaging system, using different contrast settings, differences in field of view selection, etc.

Since the presently acquired image data can be transformed to essentially any desired respiratory state after acquisition, the time required for imaging may be reduced, and/or the selection of a suitable respiratory state to image can be postponed to after the imaging procedure. For example, if it becomes apparent that in the inhale phase there is better separation between a treatment target and organs-at-risk, e.g. in a radiotherapy planning, this respiratory state can be chosen for use in planning and a gated treatment even if the raw data does not correspond to imaging of this exact state. This offers substantial freedom to the clinician planning the procedure without increasing the burden on the imaging workflow. If the clinician decides, in this example, that the exhale phase still offers better advantages for treatment, e.g. since it may be more stable to maintain and/or since a patient naturally can dwell longer in this state, the option remains open to use that phase for planning and treatment by a simple selection of a different transformation setting. Similarly, the same approach can be used to select a mid-position (e.g. generate an image representation thereof), i.e. a visualization of the time-averaged position of the treatment target, e.g. tumor.

While it is mentioned that the acquired data may be first reconstructed 111 into an image and then transformed 112 using previously gathered registration information, it is to be noted that the other approach discussed hereinabove (not necessarily mutually exclusive though), the registration information 110 that is already available, e.g. in the form of a motion model, may be used to simulate a suitable phase offset 109 for different k-space lines (trajectories, trajectory segments) to transform each acquired k-space line (with its associated respiratory parameter or parameters indicative of the crossing of center of k-space) to a respiratory state of interest. Thus, while the data to reconstruct an image may be collected over a non-negligible time window, each separate k-space line may be easily transformed to put the entire reconstruction into a desired breathing state by determining a suitable phase offset for that line. It is to be noted that this allows for a very efficient transformation process, since it only requires that a phase value is added (or subtracted) to each line in the Fourier space. For example, for each line, the respiratory parameter(s) associated with the crossing of the center of k-space (or closest point to the center for that line) may be used to look up a current state in the motion model, whereas a desired output state defines a second, target (reference), state in the motion model.

Alternatively, or additionally (e.g. for compensating residual motion), a registration technique as known in the art may be applied as well to reconstructed images, using such predetermined motion model from a previous image sequence. The previously gathered registration information may be obtained in a different imaging session, possibly even on a different imaging modality or may relate to different images acquired in the same imaging session, e.g. a 4D-MRI scan of sufficient temporal resolution. The previously gathered imaging information (earlier or same session) is preferably annotated with sufficiently accurate respiratory information such that a motion model determined by registration techniques from this previously gathered imaging data can be correlated with the respiratory parameter(s), e.g. such that a transformation from any respiratory state to any other respiratory state can be easily determined.

Without limitation to this illustrative approach, a vector field difference between these two states may be easily calculated, and an average displacement over the k-space line at line can be easily determined. Using a known displacement to phase relationship (e.g. determined from the acquisition parameters), the phase to be added for that line may be determined. Even though this approach essentially only accounts for displacements, it is to be noted that this displacement can vary over the k-space lines, such that the effect of a deformable field (e.g. non-rigid transformation) can be approximated. Furthermore, the acquisition sequence can be tuned to take this simple registration process into account, e.g. to use (without limitation thereto) a radial k-space sampling pattern to better approximate concentric displacements of chest and/or abdominal anatomy due to breathing by phase offsetting of the radial lines. Other transformations in k-space to determine for and apply to each line separately can also be considered, such as an interpolation to increase and/or decrease overall bandwidth, e.g. to better account for contraction (changes in scale across each line).

For example, when the respiratory state of each acquisition within an MRI exam is accurately determined, in accordance with embodiments of the present invention, the information gathered for one scan (or series) can also be leveraged for other scans. For example, motion information from a 4D-MRI sequence can be used for other scans as well. Such 4D-MRI sequence may be used to determine deformation vector fields (or another type of motion model), associated with the corresponding breathing state information. Another image acquisition, e.g. an MRI image with different contrast properties, may be transformed in accordance with this motion model, to obtain an approximation of a 4D sequence (or an image of, generally, any desired point in the breathing cycle different from the one associated with the acquired image being transformed). Thus, a 4D breath cycle image series can be obtained for any desired contrast setting, e.g. a 4D diffusion weighted image (DWI), T2-weighted 4D scans, or even 4D-spectroscopic scans. On the other hand, it can also be used to speed up the acquisition of a specific 4D-MRI scan and use the information from other scans to estimate the information that was not captured with the fast 4D-MRI acquisition. A relatively fast sequence can be used to characterize the temporal dynamics of the respiratory cycle, and this information can thus be used to obtain any desired respiratory state and/or a full 4D representation for another acquired image, which potentially takes substantially longer to acquire. It is also to be noted that such time intensive image acquisition is not necessarily gathered at exactly the same point in the respiratory cycle, since individual k-space lines (or trajectories, or trajectory segments) can be adjusted, at least approximatively, to a reference point by using the phase adjustment discussed hereinabove.

It is also an advantage that the phase adjustment technique discussed hereinabove can be used without requiring the triggering approach discussed hereinabove, but may also be used in combination, e.g. such that this adaptive triggering (or otherwise adjusting of the properties of the sequence to be executed to time the center of k-space to the target time point in the breath cycle) can prospectively prevent large deviations in the image being acquired from the anatomy in the intended breath phase to be visualized and the phase adjustment can retrospectively compensate for smaller deviations, e.g. to further improve the quality of the reconstructed image. It is also to be noted that the latter is particularly easy to perform and/or requires little computational resources.

A method in accordance with embodiments of the present invention may comprise reconstructing 111 the acquired k-space data to obtain one or more (spatial; real space) images, e.g. using a (inverse) Fourier transform, e.g. a Fast Fourier Transform, and/or another tomographic reconstruction technique known in the art. The breathing state (e.g. respiratory parameter or parameters *P(t_{c}),* and/or an abstraction thereof) that is determined 104 to correspond with the point in time *t_{c}* of sampling the center of k-space (e.g. in 2D or 3D, e.g. at the center of the k-space matrix) may be associated with the reconstructed image, e.g. by annotating 113 the reconstructed image to indicate the corresponding breathing phase. The point in time representative for the reconstructed image may correspond to the center of k-space sampling of the most central line (e.g. when a row-by-row sampling is used), or to a average of the center of k-space sampling for all lines that go through the k-space matrix center (e.g. for radial sampling). Likewise, images obtained by a reconstruction of phase-adjusted data 109 and/or by applying another registration technique 112 as discussed hereinabove may be annotated 113 with respiratory state information as well.

It is an advantage that the breathing state associated with an image can be accurately determined and added as side-information to the image, e.g. incorporated in metadata, such that this information can be taken into account in analysis of the image, e.g. by a clinician and/or by an automated algorithm, and/or in further processing applied to the image. For example, the respiratory state may be characterized by at least one respiratory parameter associated with the point of time determined for the image, e.g. which may comprise (a) value(s) such as a value indicative of a point in time (e.g. a phase value) in the breathing cycle and/or other parameters of the breathing, e.g. an amplitude value. The respiratory state may also be represented by a breathing phase indicator, e.g. in which the breathing cycle is split into a discrete number of phases, e.g. four or ten phases (without limitation to these numbers). Once the respiratory state or phase of a reconstructed image is determined, this information may be added as an annotation, such as in a header of a DICOM file storing the image, e.g. in a DICOM tag. This way, a treatment planning system, delineation program, other processing software and/or a user can determine to which breathing state an image corresponds, e.g. to determine if images are acquired in a specific or in the same respiratory state/phase and therefore are suitable for the intended processing. This information can also be used to determine in which respiratory state/phase new images should be acquired to allow a good comparison to earlier acquired images, e.g. in a follow-up or therapy session, such as in daily imaging on an MR-Linac system.

It is also an advantage that the point at which each line (or trajectory segment, e.g. row, radial line, straight line segment in a zig-zag pattern, spiral line ...) crosses through (or passes as near as possible to) the center of k-space may be determined to associate a specific breathing state with each such line (or line segment). For example, the MRI data may be acquired for the same k-space line (or trajectory segment) a plurality of times, generally at different points of the breath cycle, e.g. in an oversampling approach. Thus, the step of reconstructing 111 an image may comprise selecting 114 a set of k-space data such that the entire raw data (k-space) matrix (or at least the part thereof to reconstruct) is filled by the union of the selected data, in which the selected lines are sufficiently close to the intended respiratory point (or phase-corrected to that point).

In an embodiment in accordance with embodiments of the present invention, a (same) line (or trajectory, or trajectory segment) may be acquired repeatedly 115, e.g. to obtain oversampled data. Since the respiratory state corresponding to each sampled k-line can be accurately determined, and each sample may be generally obtained for a different position along the breathing cycle, the reconstruction can determine a k-space matrix to reconstruct for a predetermined breathing state by selecting 114 suitable k-space samples to fill the matrix, e.g. selecting the lines or trajectories (segments) that are closest to the desired respiratory state, by interpolating between the closest respiratory states and/or by using a weighted average or other aggregation technique to take a distance measure between the desired respiratory state and the respiratory state determined for the (e.g. each) line into account. Thus, retrospectively, oversampled data with respiratory state annotation may be used to reconstruct any desired point in the breathing cycle. The same data for the same k-space matrix (2D or 3D) can be acquired multiple times during a single respiratory cycle. For example, a 2D image or part of a 3D k-space that takes 200 ms to acquire can be sampled 25 times during a respiratory cycle of typically about 5 seconds. If the same part of k-space is sampled during a single respiratory cycle and change which region is acquired for the next respiratory cycle, any respiratory state can be reconstructed during reconstruction. Even though more data may need to be collected when using an oversampling technique, the advantages may outweigh this aspect. For example, not only can any desired respiratory state be accurately reconstructed, more than one line may be sampled that is sufficiently close to any desired state to use in any state reconstruction (e.g. by averaging, weighted averaging or the like), thus improving signal to noise characteristics. This may even be further improved by combining with the phase offsetting technique discussed hereinabove. The acquisition sequence may also be tuned to take this oversampling and flexible reconstruction into account. For example, the entire k-space may be sparsely sampled in each breath cycle to create a completely sampled k-space matrix over a plurality of cycles, only one or a few k-space parts may be sampled in each breath cycle, and/or other variations may be considered to balance the total acquisition time to the desired level of oversampling of each or specific points in the respiratory cycle.

Commonly, e.g. in current radiotherapy practice, a predetermined breathing phase is selected to be imaged and used in therapy planning, often in combination with aiming to align the radiotherapy delivery to this breathing phase. Alternatively, a relatively small number of images is acquired for different respiratory phases to offer more flexibility in planning.

However, the mid-position would often be the most suitable for treatment, in a statistical sense, even though this is in practice not often used, e.g. relying instead on a single respiratory phase (e.g. selected from a 4D-CT series) as reference for planning and treatment. Instead of a single respiratory phase, the internal target volume (ITV) may also be commonly relied upon, which uses a time-integrated image (i.e. the sum or, when appropriately normalized, the average of the phase images in a 4D scan) to define the tumor volume to treat. Clearly, the latter may also result in irradiation of a larger volume than strictly necessary and, thus, potentially unnecessary damage to healthy tissue in the vicinity of the tumor.

By offering a convenient approach to accurately characterize the respiration for a plurality of images, e.g. possibly by a relatively large number of reconstructions and/or by allowing any arbitrary point in the breathing cycle to be reconstructed (e.g. using the phase correction, oversampling and/or taking a motion model into account determined from pre-acquired imaging data), an algorithm can be used to determine the most appropriate breathing phase for therapy delivery. This can be based on conventional modeling, a combination of expert knowledge and algorithm design and/or machine learning techniques. For example, since a large amount of image data over the entire breath cycle can be obtained for each patient/test subject, a machine learning algorithm can be trained to select the best candidate breathing phase for therapy, e.g. based on supervised learning taking a clinician's decision as output to train and/or by defining an objective function constructed to take relevant features into account, such as separation between treatment target and organs at risk, dwell time of the target in a stable position, and/or other such potentially relevant factors. Therefore, from the gathered data, it can be determined which respiratory phase from a 4D-CT, 4D-MRI or 4D-PET (without restriction to these illustrative modalities) is most representative for the patient. It is also to be noted that embodiments of the present invention may allow for a natural aggregation of information from wider time windows and/or over multiple breathing cycles to characterize a specific point (and a plurality of such points) in the breath cycle, e.g. by using oversampling, such that the images thus acquired (and reconstructed) may offer a better representation of the average conditions in the body at that point in the breath cycle than a conventional 4D-CT and/or 4D-MRI.

Whereas conventionally respiratory motion can be characterized by such a 4D-CT or 4D-MRI scan, the acquisition time may be insufficient to fully characterize the respiratory motion, e.g. only offering a few acquired and reconstructed phases. Nonetheless, the required acquisition time may often be relatively long in comparison with other scans, e.g. in the order of 3 to 10 minutes. The mid-position, defined by the time-averaged position of the tumor and/or organs-at-risk, may differ when determined from a more detailed 4D imaging procedure (e.g. in terms of temporal resolution and/or acquired over a longer acquisition time window) than when determined using such conventional 4D-CT or 4D-MRI scan. In accordance with embodiments of the present invention, the respiratory motion can be characterized in detail, such that also a mid-position of a volume of interest in the body, e.g. the time-averaged position of a tumor and/or organ(s) at risk over a breath cycle, may be accurately determined. This may be achieved by prospectively acquiring MRI data at or near time points that are representative of the mid-position (e.g. determined by continually analyzing the respiratory signal) and then (optionally) registering different mid-positions to each other, e.g. acquired at different time points (e.g. in different breath cycles), e.g. potentially also using the phase adjustment approach discussed hereinabove. It is to be noted that data may also be acquired without tuning the time points to the presumed mid-point state, e.g. if sufficient sampling over the breathing cycle (and potentially over a plurality of breathing cycles) is achieved, e.g. such that suitable data can be selected and/or appropriately weighted retrospectively. An image representative of the mid-position may also be determined by weighting reconstructed images from the different time points, e.g. taking the actual breathing state at its acquisition time into account and/or the relative position of the target volume in relation to the other mid-position scans (e.g. to a selected reference scan). The mid-position information may even be updated during an MRI-guided treatment, e.g. using an MR-Linac combination, e.g. to get a daily (or other frequency) update of the mid-position.

In a second aspect, the present invention relates to a computer-program product as defined in claim 13.

In a third aspect, the present invention relates to a magnetic resonance imaging system comprising a device for respiratory monitoring of a subject's respiration during a magnetic resonance imaging session as defined in claim 1. Fig 2 shows an illustrative magnetic resonance imaging system 10 in accordance with embodiments of the present invention.

For example, the system may comprise a processor, data storage memory, input(s), output(s), a user interface and/or other means generally known for performing a method as discussed hereinabove, e.g. when programmed and/or configured accordingly. Thus, the system may comprise a computer 20 and a computer-program product in accordance with the second aspect of the present invention (i.e. adapted to be executed by the computer 20). Additionally or alternatively, the system may comprise hardware specifically designed and/or configured to perform a method in accordance with embodiments of the first aspect of the present invention, e.g. comprising an application specific integrated circuit and/or configured field-programmable gate array to perform a method in accordance with the first aspect of the present invention.

The device 10 is comprised in a magnetic resonance imaging system, in accordance with the present invention.

The device 10 comprises an input 11 for receiving sensor data and determining at least one respiratory parameter therefrom to continually monitor the respiration of the subject during the MRI session. The input may comprise a sensor or plurality of sensors (e.g. a spirometer, a camera, and/or another sensing device suitable for acquiring information from which the respiration of the subject can be characterized), and/or is adapted to be operably connected to such sensor(s). The device may thus also comprise a sensor input processor, e.g. integrated in a multi-purpose processor 14, to process the received sensor data and determine the respiratory parameter(s) therefrom. Examples of suitable sensors and parameters to be derived therefrom were discussed hereinabove, without limitation to these examples.

The device 10 may also comprise a feedback output 12 for providing sensory feedback to the subject during the MRI session to guide the breathing behavior of the subject toward a predetermined breathing pattern. The sensory feedback may be visual, auditive, tactile and/or delivered by another sensory feedback mechanism to the subject, or a combination of different sensory modalities. Therefore, the feedback output may comprise a monitor, a projector, an indicator light, headphones, a speaker, an actuator, a vibrating element, and/or another suitable device for delivering sensory feedback, or a combination of means for delivering different sensory modalities. Alternatively, the output may be adapted to operably connect to such output device(s). The device may thus also comprise a feedback processor, e.g. integrated in a multi-purpose processor 14, for determining a suitable feedback signal(s) to provide via the output 12 based on the determined respiratory parameter(s) determined from the received input 11 in relation to the predetermined beathing pattern to achieve.

The device 10 comprises a data input 13 for receiving magnetic resonance imaging data comprising k-space values along at least one trajectory segment in k-space, in one or more acquisitions, e.g. to sample and fill a k-space matrix or matrices to reconstruct one or more images from, e.g. by filling a plurality of lines and/or trajectory segments through the matrix until sufficient (or all) values in the matrix are sampled to allow MRI image reconstruction.

The device 10 comprises a processor 14 adapted for determining a point in time when the center of the k-space matrix and/or the nearest point of the (or each) k-space trajectory segment to the center of the k-space matrix is sampled for the received magnetic resonance imaging data, e.g. for each trajectory segment in k-space. For example, the device may comprise a computer, an application specific integrated circuit and/or configurable digital processing hardware for performing the processing tasks discussed hereinbelow, e.g. method steps of a method in accordance with the first aspect of the present invention as discussed hereinabove. It will be understood that the schematic representation of the device in Fig 2 does not necessarily correspond to a physical arrangement of components of the device, e.g. functions of the reconstructor 15 may be implemented by the processor as well. Likewise, functionality of the processor 14 as discussed herein may be distributed over several processing units, e.g. in a multi-core processor, a graphical processing unit processor, a cell processor, a dedicated configured or specifically designed hardware device (e.g. ASIC, FPGA, ...), a combination of one or more of said generally known components, or even distributed over several computer systems, e.g. executed on a computer cluster.

The processor 14 is furthermore adapted to determine the at least one respiratory parameter for said point in time using the at least one respiratory parameter monitoring the respiration.

The processor 14 is adapted to use the monitored at least one respiratory parameter to determine prospectively the point in time when the respiration will correspond to a reference respiratory state, and, via a controller output 19, control at least one acquisition parameter of the acquisition of the or each trajectory segment such that said point in time determines the time of sampling the center of the k-space matrix and/or the nearest point of the k-space trajectory segment to the center of the k-space matrix. For example the controller output 19 may be adapted to provide a trigger signal to the magnetic resonance imaging system to initiate the acquisition of data for a k-space trajectory segment at a suitable time determined such that the time of acquiring the center of k-space data, or the closest point on the trajectory segment thereto, coincides with the reference respiratory state. Additionally or alternatively, another acquisition parameter or parameters may be controlled to affect the time of sampling the nearest point to the center of k-space, e.g. by adjusting field gradient and/or pulse configurations. As already mentioned, the device may be integrated in an MRI system (or an operator terminal thereof), such that the output may merely refer to an internal state of the device, e.g. in a data memory, that is accessible by a control system or program of the MRI system to control the MRI system accordingly.

The device may comprise a data storage 16. The data storage may be integrated in the device, or may refer to an interface for accessing an external data storage, e.g. via a data communication network. For example, the data storage unit may comprise a data storage disk, a hard drive, a database, a network drive or any other suitable means for storing digital information. The processor may be adapted to retrieve the reference respiratory state from the data storage.

The device may also comprise a user interface 17, e.g. which may comprise a display monitor, a mouse, a keyboard and/or one or more similar human interface devices known in the art. The user interface may be adapted to control the processes discussed hereinabove and below, e.g. to configure the breathing monitoring, set a target respiratory state for feedback, start and/or stop an image acquisition, start a reconstruction and/or other such actions. The processor may be adapted to receive the reference respiratory state from a user via the user interface as target for the prospective triggering and/or sequence adjustment discussed hereinabove, and as target for the retrospective phase adjustment and/or reconstruction discussed hereinbelow. The user interface 17 may be adapted for interacting with an operator, e.g. a healthcare professional, such as a medical doctor, a nurse, a radiotherapist, an imaging technologist or the like. For example, the user interface may be used by the operator to control the device and/or observe the respiration as monitored by the device.

The processor 14 may be adapted to use the monitored at least one respiratory parameter to determine a plurality of points in time when the respiration will correspond to the reference respiratory state for respectively acquiring a plurality of trajectory segments such that each determined point in time determines the time of sampling the center of the k-space matrix and/or the nearest point on each k-space trajectory segment to the center of the k-space matrix.

The processor 14 may be adapted to control the at least one acquisition parameter for each of the plurality of trajectory segments taking a predetermined time window for the collective acquisition of the plurality of trajectory segments into account, and to adaptively apply a sparse sampling and/or compressed sensing technique toward and/or past the predetermined time window.

Additionally to the prospective control of the acquisition(s) taking the respiratory monitoring into account, the processor 14 is adapted to retrospectively, i.e. after the acquisition(s), compare the determined at least one respiratory parameter corresponding to said time of sampling of the center of, or the nearest point on the trajectory segment to the center of, the k-space matrix to a predetermined reference value, corresponding to the reference respiratory state, to determine a phase offset from this comparison and to apply the phase offset to the k-space values obtained for the trajectory segment to compensate for a translation in real space due to respiratory motion thus determined from the comparison.

The processor 14 may also be adapted to retrieve previously acquired images of said subject from the data storage 16, in which respiratory information is associated with each of said previously acquired images. The processor may be adapted to register the previously acquired images and model transformations obtained by said registration as function of the respiratory information to obtain a motion model. The processor may be adapted to determine the phase offset to compensate for a translation in real space due to respiratory motion using said motion model.

Alternatively, the processor 14 may be adapted to retrieve such registration data or motion model directly from the data storage, e.g. where it was stored by an external registration and/or motion modeling system.

The previously acquired images may relate to images obtained in a previous imaging session, e.g. possibly using a different imaging modality. Alternatively, the previously acquired images may be obtained previously in the same MRI imaging session and stored for said registration and/or motion modeling (e.g. after reconstruction).

The device may comprise a reconstructor 15 for reconstructing the received magnetic resonance imaging data and/or the phase-corrected magnetic resonance imaging data into at least one tomographic image. The reconstructor may also be integrated in the processor 14, e.g. the processor may be adapted for reconstructing images. The reconstructor 15 may furthermore be adapted to annotate the reconstructed tomographic image with a respiratory state that is determined by the at least one respiratory parameter that is determined, by the processor, to correspond with said point in time of sampling the center of k-space and/or said predetermined reference value used for determining the phase offset to adjust the magnetic resonance imaging data used for the reconstruction.

The reconstructor 15 may also be adapted to select a (proper) subset (e.g. non-empty and not comprising all acquired trajectory segments) of the magnetic resonance imaging data such as to fill the k-space matrix with the selected magnetic resonance imaging data for reconstruction, in which k-space trajectory segments are selected based on a relation of the at least one respiratory parameter determined for each k-space trajectory segment with respect to a reference respiratory state to reconstruct, e.g. selecting the nearest candidate, averaging a plurality of close candidates, using a weighted average taking a distance metric between the determined respiratory parameter and the reference into account, and/or another similar strategy.

The processor 14 may also be adapted to control the acquisition of the magnetic resonance imaging data by repeatedly acquiring a same k-space trajectory segment at different points in the respiratory cycle (e.g. actively varying the sampled point in the respiratory cycle and/or by simply uncorrelated sampling with respect to the respiratory cycle). The reconstructor 15 may be adapted to reconstruct images for different respiratory states by a corresponding selection from, interpolation between and/or weighting of different sets of data acquired for said same k-space trajectory segment (or rather, also different trajectory segments, e.g. lines, may be sampled repeatedly in the discussed manner such that sufficient data becomes available to reconstruct a complete 2D or 3D image for any arbitrary point in the respiratory cycle). The phase adjustment technique discussed hereinabove may also be used to adjust data of k-space trajectory segments to the desired respiratory state for reconstruction. For example a selection of one or more trajectory segment candidates may select the candidate(s) that were acquired sufficiently close in time to the target respiratory state and this selection may be phase-adjusted to the desired state. If more candidates are thus selected and phase-adjusted for the same trajectory segment, the values may be combined by averaging, weighted averaging (e.g. weighted by an inverse distance between the original respiratory parameter or parameters associated with the data before phase correction and the target respiratory parameter/s of the target respiratory state), and/or another suitable aggregation technique. Thus, this approach may be applied to different trajectory segments until the k-space matrix is filled or at least has enough data to enable reconstruction.

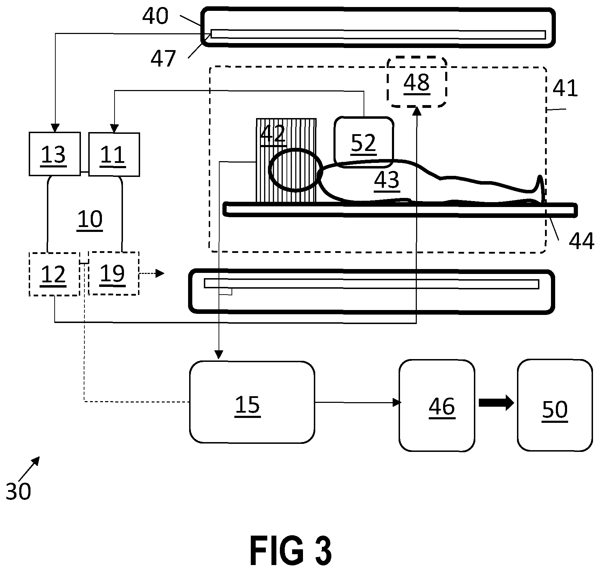

Fig 3 schematically shows a magnetic resonance imaging system 30, in accordance with embodiments of the present invention. The MRI system may comprise a primary magnet assembly 40, which defines an examination zone 41, e.g. the examination zone may be formed by a volume where the magnetic field conditions, as substantially created and controlled by the magnet assembly, are suitable for magnetic resonance imaging. The examination zone may thus correspond to (at least a usable portion of) the volume enclosed by a magnet bore of the system (without limitation, e.g. principles of the present invention equally apply to open bore systems and other, less frequently used, magnet assembly configurations).

A subject, e.g. a patient, to be examined 43 may, in use of the system, be positioned on a patient couch 44 in the examination zone. The primary magnet assembly may comprise magnet windings, e.g. coaxial (e.g. superconductive) windings, to generate a stationary uniform magnetic field in the examination zone. The examination zone may be a cylindrical volume encompassed by these magnet windings.

The system may comprise a reconstructor 15 to reconstruct magnetic resonance image(s), e.g. tomographic MRI images, from magnetic resonance signals acquired by the system in use. The reconstructed images may be provided via an output 46 for viewing, processing or storage. The output (directly or indirectly, e.g. via an intermediate storage) may be received by a magnetic resonance imaging workstation 50, where a medical worker can interact with the received images, e.g. to view the images, to manipulate the images, to diagnose a condition of the patient and/or to plan a therapy. The workstation 50 may, optionally, also comprise the reconstructor 15.

Auxiliary equipment, such as an auxiliary RF transmit and/or receive coil assembly 42 may, in use, be place in the examination zone to acquire magnetic resonance signals from a specific part of the subject's body. Auxiliary coil configurations may be used to acquire signals from specific body parts or for specific use cases, while, typically, signals may also (additionally or alternatively) be received by receiver coils 47 directly integrated in the housing of the primary magnet assembly. The primary magnet assembly (or, generally, the magnetic resonance imaging system) typically also comprises gradient coils to create (controllable) spatial gradients of the magnetic field inside the examination area and/or to create RF excitation pulses.

The system may comprise a respiratory sensor 52 for acquiring respiratory data indicative of the breathing of the subject 43. The respiratory sensor may comprise a breathing belt sensor, a spirometer, an observation camera (with a suitable video processing system to derive respiratory information from the camera observation of the subject), and/or another type of sensor or sensors suitable for obtaining data from which relevant respiratory information can be obtained, e.g. as already discussed in detail hereinabove in relation to a method in accordance with embodiments of the present invention.

A device 10 in accordance with embodiments of the present invention may be integrated in the workstation 50, or elsewhere into the magnetic resonance system 30 (or the functionality of the device as discussed may be distributed between the MRI system and the workstation). The device may be configured to receive its sensor input 11 from the respiratory sensor 52. It may also be configured to receive its data input from (a) receive coil(s) 47 (or an intermediate signal processor or controller) of the MRI system.

A controller output 19 of the device may connect to a system controller of the MRI system, e.g. such as to control the data acquisition of the system, e.g. to trigger an acquisition and/or control other acquisition parameters, such as gradient, pulse and/or data collection configurations.

The MRI system may also comprise a sensory feedback system 48 to provide sensory feedback to the subject, e.g. to guide the subject's breathing to a desired pattern. The feedback system may comprise a visual, auditive, tactile or other sensory output that can be perceived by the subject, e.g. a projector, a screen, headphones, speakers, a vibrating element, an actuator and/or other such means, or a combination thereof. Reference is also made to the discussion hereinabove regarding feedback systems in relation to a method in accordance with embodiments of the present invention.

Other features, or details of the features described hereinabove of a magnetic resonance imaging system (resp. computer-program product) in accordance with embodiments of the present invention shall be clear in view of the description provided hereinabove relating to a method in accordance with embodiments of the present invention, and/or vice versa.

## Claims

1. A magnetic resonance imaging system comprising a device (10) for respiratory monitoring of a subject's respiration during a magnetic resonance imaging session, the device comprising:
a sensor input (11) for receiving sensor data and determining at least one respiratory parameter therefrom to continually monitor the respiration of the subject during the magnetic resonance imaging session,
a data input (13) for receiving magnetic resonance imaging data comprising k-space values along at least one trajectory segment in a k-space matrix,
a controller output (19), and
a processor (14), wherein the processor (14) is configured to perform a method (100) for respiratory monitoring of a subject's respiration during a magnetic resonance imaging session, the method comprising:
acquiring (101) magnetic resonance imaging data comprising k-space values along at least one trajectory segment in a k-space matrix,
monitoring (102) the respiration of the subject by measuring one or more respiratory parameters,
prospectively determining a point in time when the respiration will correspond to a reference respiratory state, and control at least one acquisition parameter of an acquisition of the or each trajectory segment such that said point in time when the respiration will correspond to a reference respiratory state determines the time of sampling of the nearest point of the k-space trajectory segment to the center of the k-space matrix by using the monitored at least one respiratory parameter,
**characterised by** the method further comprising determining (103) a point in time when the nearest point of the k-space trajectory segment to the center of the k-space matrix was sampled in acquiring (101) the magnetic resonance imaging data,
determining (104) the at least one respiratory parameter for said point in time using said monitoring (102) of the respiration, and
retrospectively comparing the determined at least one respiratory parameter corresponding to said time of sampling of the nearest point on the trajectory segment to the center of the k-space matrix to a predetermined reference value corresponding to the reference respiratory state, to determine a phase offset from this comparison and to apply the phase offset to the k-space values obtained for the trajectory segment to compensate for a translation in real space due to respiratory motion as determined from said comparison.

2. The system of claim 1, wherein said processor (14) is adapted to use the monitored at least one respiratory parameter to determine a plurality of points in time when the respiration will correspond to the reference respiratory state for respectively acquiring a plurality of trajectory segments such that each determined point in time determines the time of sampling of the nearest point on each k-space trajectory segment to the center of the k-space matrix.

3. The system of claim 2, wherein said processor (14) is adapted to control the at least one acquisition parameter for each of said plurality of trajectory segments taking a predetermined time window for the collective acquisition of the plurality of trajectory segments into account, and to adaptively apply a sparse sampling and/or compressed sensing technique toward and/or past the predetermined time window.

4. The system of any one of the preceding claims, comprising a data storage (16), wherein said processor (14) is adapted to retrieve previously acquired images of said subject from the data storage (16), in which respiratory information is associated with each of said previously acquired images, the processor being furthermore adapted to register the previously acquired images and model transformations obtained by said registration as function of the respiratory information to obtain a motion model and to determine the phase offset to compensate for a translation in real space due to respiratory motion using said motion model.

5. The system of any of the previous claims, comprising a reconstructor (15) for reconstructing the phase-corrected magnetic resonance imaging data into at least one tomographic image.

6. The system of claim 5, wherein said reconstructor (15) is adapted to annotate the reconstructed tomographic image with a respiratory state that is determined by the at least one respiratory parameter that is determined to correspond with said point in time of sampling the nearest point on each k-space trajectory segment to the center of the k-space matrix, by said predetermined reference value used for determining the phase offset to adjust the magnetic resonance imaging data used for the reconstruction.

7. The system of claim 5 or 6, wherein said reconstructor (15) is adapted to select a proper subset of the magnetic resonance imaging data such as to fill the k-space matrix with the selected magnetic resonance imaging data for reconstruction, in which k-space trajectory segments are selected based on a relation of the at least one respiratory parameter determined for each k-space trajectory segment with respect to a reference respiratory state to reconstruct.

8. The system of any of the claims 5 to 7, wherein said processor (14) is adapted to control the acquisition of the magnetic resonance imaging data by repeatedly acquiring a same k-space trajectory segment at different points in the respiratory cycle in an oversampling strategy, wherein said reconstructor (15) is adapted to reconstruct images for different respiratory states by a corresponding selection from, interpolation between and/or weighting of data acquired for said same k-space trajectory segment.

9. The system of any of the previous claims, comprising a feedback output (12) for providing sensory feedback to the subject during the magnetic resonance imaging session to guide the breathing behavior of the subject toward a predetermined breathing pattern.

10. The system of any of the previous claims, wherein the sensor data is syncronized to the magnetic resonance imaging data.

11. The system of any of the previous claims, wherein the processor is configured to determined the point in time when the nearest point of the or each k-space trajectory segment to the center of the k-space matrix at least partially using the at least one acquisition parameter.

12. A method (100) for respiratory monitoring of a subject's respiration during a magnetic resonance imaging session, the method comprising:
acquiring (101) magnetic resonance imaging data comprising k-space values along at least one trajectory segment in a k-space matrix,
monitoring (102) the respiration of the subject by measuring one or more respiratory parameters,
prospectively determining a point in time when the respiration will correspond to a reference respiratory state, and control at least one acquisition parameter of an acquisition of the or each trajectory segment such that said point in time when the respiration will correspond to a reference respiratory state determines the time of sampling of the nearest point of the k-space trajectory segment to the center of the k-space matrix by using the monitored at least one respiratory parameter,
**characterized by** the method further comprising determining (103) a point in time when the nearest point of the k-space trajectory segment to the center of the k-space matrix was sampled in acquiring (101) the magnetic resonance imaging data,
determining (104) the at least one respiratory parameter for said point in time using said monitoring (102) of the respiration, and
retrospectively comparing the determined at least one respiratory parameter corresponding to said time of sampling of the nearest point on the trajectory segment to the center of the k-space matrix to a predetermined reference value corresponding to the reference respiratory state, to determine a phase offset from this comparison and to apply the phase offset to the k-space values obtained for the trajectory segment to compensate for a translation in real space due to respiratory motion as determined from said comparison.

13. A computer-program product comprising instructions to cause the magnetic resonance imaging system of claim 1 to execute the method steps of claim 12.

## Patentansprüche

1. Magnetresonanztomographiesystem, das eine Vorrichtung (10) zur Überwachung der Atmung eines Probanden während einer Magnetresonanztomographie-Untersuchung umfasst, wobei die Vorrichtung Folgendes umfasst:
einen Sensoreingang (11) zum Empfangen von Sensordaten und zum Bestimmen mindestens eines Atemparameters daraus, um die Atmung des Probanden während der Magnetresonanztomographie-Untersuchung kontinuierlich zu überwachen,
einen Dateneingang (13) zum Empfangen von Magnetresonanztomographiedaten, die k-Raum-Werte entlang mindestens eines Trajektoriensegments in einer k-Raum-Matrix umfassen,
einen Steuereinheitsausgang (19) und
einen Prozessor (14), wobei der Prozessor (14) so konfiguriert ist, dass er ein Verfahren (100) zur Überwachung der Atmung eines Probanden während einer Magnetresonanztomographie-Untersuchung durchführt, wobei das Verfahren Folgendes umfasst:
Erfassen (101) von Magnetresonanztomographiedaten, die k-Raum-Werte entlang mindestens eines Trajektoriensegments in einer k-Raum-Matrix umfassen,
Überwachen (102) der Atmung des Probanden durch Messen eines oder mehrerer Atemparameter,
prospektives Bestimmen eines Zeitpunkts, an dem die Atmung einem Referenz-Atemzustand entspricht, und Steuern mindestens eines Erfassungsparameters einer Erfassung des oder jedes Trajektoriensegments so, dass der Zeitpunkt, an dem die Atmung einem Referenz-Atemzustand entspricht, den Abtastzeitpunkt des nächstgelegenen Punktes des k-Raum-Trajektoriensegments zum Zentrum der k-Raum-Matrix unter Verwendung des überwachten mindestens einen Atemparameters bestimmt,
**dadurch gekennzeichnet, dass** das Verfahren weiter Bestimmen (103) eines Zeitpunkts umfasst, an dem der nächstgelegene Punkt des k-Raum-Trajektoriensegments zum Zentrum der k-Raum-Matrix beim Erfassen (101) der Magnetresonanztomographiedaten abgetastet wurde,
Bestimmen (104) des mindestens einen Atemparameters für den Zeitpunkt unter Verwendung der Überwachung (102) der Atmung, und
retrospektives Vergleichen des bestimmten mindestens einen Atemparameters, der dem Abtastzeitpunkt des nächstgelegenen Punktes auf dem Trajektoriensegment zum Zentrum der k-Raum-Matrix entspricht, mit einem vorbestimmten Referenzwert, der dem Referenz-Atemzustand entspricht, um aus diesem Vergleich eine Phasenverschiebung zu bestimmen und die Phasenverschiebung auf die für das Trajektoriensegment erhaltenen k-Raum-Werte anzuwenden, um eine Translation im realen Raum aufgrund von Atembewegung, wie aus dem Vergleich bestimmt, zu kompensieren.

2. System nach Anspruch 1, wobei der Prozessor (14) so ausgelegt ist, dass er den überwachten mindestens einen Atemparameter verwendet, um eine Vielzahl von Zeitpunkten zu bestimmen, zu denen die Atmung dem Referenz-Atemzustand entspricht, um jeweils eine Vielzahl von Trajektoriensegmenten zu erfassen, so dass jeder bestimmte Zeitpunkt den Abtastzeitpunkt des nächstgelegenen Punktes auf jedem k-Raum-Trajektoriensegment zum Zentrum der k-Raum-Matrix bestimmt.

3. System nach Anspruch 2, wobei der Prozessor (14) so ausgelegt ist, dass er mindestens einen Erfassungsparameter für jedes der Vielzahl von Trajektoriensegmenten unter Berücksichtigung eines vorbestimmten Zeitfensters für die gemeinsame Erfassung der Vielzahl von Trajektoriensegmenten steuert und eine adaptive, spärliche Abtastung und/oder Compressed-Sensing-Technik gegenüber und/oder nach Ablauf des vorbestimmten Zeitfensters anwendet.

4. System nach einem der vorstehenden Ansprüche, das einen Datenspeicher (16) umfasst, wobei der Prozessor (14) dazu ausgelegt ist, zuvor erfasste Bilder des Subjekts aus dem Datenspeicher (16) abzurufen, wobei jedem der zuvor erfassten Bilder Ateminformationen zugeordnet sind, wobei der Prozessor weiter dazu ausgelegt ist, die zuvor erfassten Bilder zu registrieren und die durch die Registrierung erhaltenen Transformationen als Funktion der Ateminformationen zu modellieren, um ein Bewegungsmodell zu erhalten und die Phasenverschiebung zu bestimmen, um eine Translation im realen Raum aufgrund der Atembewegung unter Verwendung des Bewegungsmodells zu kompensieren.

5. System nach einem der vorstehenden Ansprüche, das einen Rekonstruktor (15) zum Rekonstruieren der phasenkorrigierten Magnetresonanztomographiedaten in mindestens ein tomographisches Bild umfasst.

6. System nach Anspruch 5, wobei der Rekonstruktor (15) so ausgelegt ist, dass er das rekonstruierte tomographische Bild mit einem Atemzustand, der durch den mindestens einen Atemparameter bestimmt wird, der dem Abtastzeitpunkt des nächstgelegenen Punktes auf jedem k-Raum-Trajektoriensegment zum Zentrum der k-Raum-Matrix entspricht, mit dem vorbestimmten Referenzwert versieht, der zum Bestimmen der Phasenverschiebung verwendet wird, um die für die Rekonstruktion verwendeten Magnetresonanz-Bildgebungsdaten anzupassen.

7. System nach Anspruch 5 oder 6, wobei der Rekonstruktor (15) so ausgelegt ist, dass er eine geeignete Teilmenge der Magnetresonanztomographiedaten auswählt, um die k-Raum-Matrix mit den ausgewählten Magnetresonanztomographiedaten für die Konstruktion zu füllen, wobei k-Raum-Trajektoriensegmente auf der Grundlage einer Beziehung des mindestens einen Atemparameters, der für jedes k-Raum-Trajektoriensegment bestimmt wird, in Bezug auf einen Referenz-Atemzustand zur Rekonstruktion ausgewählt werden.

8. System nach einem der Ansprüche 5 bis 7, wobei der Prozessor (14) so ausgelegt ist, dass er die Erfassung der Magnetresonanztomographiedaten steuert, indem er wiederholt ein selbes k-Raum-Trajektoriensegment zu verschiedenen Zeitpunkten des Atemzyklus mittels einer Oversampling-Strategie erfasst, wobei der Rekonstruktor (15) so ausgelegt ist, dass er Bilder für verschiedene Atemzustände durch entsprechende Auswahl aus Interpolation zwischen und/oder Gewichtung der für dasselbe k-Raum-Trajektoriensegment erfassten Daten rekonstruiert.

9. System nach einem der vorstehenden Ansprüche, umfassend einen Rückkopplungsausgang (12) zum Bereitstellen von sensorischer Rückkopplung an den Probanden während der Magnetresonanztomographie-Untersuchung, um das Atemverhalten des Probanden in Richtung eines vorbestimmten Atemmusters zu lenken.

10. System nach einem der vorstehenden Ansprüche, wobei die Sensordaten mit den Magnetresonanztomographiedaten synchronisiert sind.

11. System nach einem der vorstehenden Ansprüche, wobei der Prozessor so konfiguriert ist, dass er den Zeitpunkt des nächstgelegenen Punkts des oder jedes k-Raum-Trajektoriensegments zum Zentrum der k-Raum-Matrix mindestens teilweise unter Verwendung des mindestens einen Erfassungsparameters bestimmt.

12. Verfahren (100) zur Überwachung der Atmung eines Probanden während einer Magnetresonanztomographie-Untersuchung, wobei das Verfahren Folgendes umfasst:
Erfassen (101) von Magnetresonanztomographiedaten, die k-Raum-Werte entlang mindestens eines Trajektoriensegments in einer k-Raum-Matrix umfassen,
Überwachen (102) der Atmung des Probanden durch Messen eines oder mehrerer Atemparameter,
prospektives Bestimmen eines Zeitpunkts, an dem die Atmung einem Referenz-Atemzustand entspricht, und Steuern mindestens eines Erfassungsparameters einer Erfassung des oder jedes Trajektoriensegments so, dass der Zeitpunkt, an dem die Atmung einem Referenz-Atemzustand entspricht, den Abtastzeitpunkt des nächstgelegenen Punktes des k-Raum-Trajektoriensegments zum Zentrum der k-Raum-Matrix unter Verwendung des überwachten mindestens einen Atemparameters bestimmt,
**dadurch gekennzeichnet, dass** das Verfahren weiter Bestimmen (103) eines Zeitpunkts umfasst, an dem der nächstgelegene Punkt des k-Raum-Trajektoriensegments zum Zentrum der k-Raum-Matrix beim Erfassen (101) der Magnetresonanztomographiedaten abgetastet wurde,
Bestimmen (104) des mindestens einen Atemparameters für den Zeitpunkt unter Verwendung der Überwachung (102) der Atmung, und
retrospektives Vergleichen des bestimmten mindestens einen Atemparameters, der dem Abtastzeitpunkt des nächstgelegenen Punktes auf dem Trajektoriensegment zum Zentrum der k-Raum-Matrix entspricht, mit einem vorbestimmten Referenzwert, der dem Referenz-Atemzustand entspricht, um aus diesem Vergleich eine Phasenverschiebung zu bestimmen und die Phasenverschiebung auf die für das Trajektoriensegment erhaltenen k-Raum-Werte anzuwenden, um eine Translation im realen Raum aufgrund von Atembewegung, wie aus dem Vergleich bestimmt, zu kompensieren.

13. Computerprogrammprodukt, das Anweisungen umfasst, um das Magnetresonanztomographiesystem nach Anspruch 1 zu veranlassen, die Verfahrensschritte nach Anspruch 12 auszuführen.

## Revendications

1. Système d'imagerie par résonance magnétique comprenant un dispositif (10) pour la surveillance respiratoire de la respiration d'un sujet au cours d'une séance d'imagerie par résonance magnétique, le dispositif comprenant :
une entrée de capteurs (11) pour recevoir des données de capteurs et déterminer au moins un paramètre respiratoire à partir de celles-ci afin de surveiller en continu la respiration du sujet pendant la séance d'imagerie par résonance magnétique,
une entrée de données (13) pour recevoir des données d'imagerie par résonance magnétique comprenant des valeurs de l'espace des k le long d'au moins un segment de trajectoire dans une matrice de l'espace des k,
une sortie de dispositif de commande (19), et
un processeur (14), dans lequel le processeur (14) est configuré pour exécuter un procédé (100) de surveillance respiratoire de la respiration d'un sujet au cours d'une séance d'imagerie par résonance magnétique, le procédé comprenant :
l'acquisition (101) de données d'imagerie par résonance magnétique comprenant des valeurs de l'espace des k le long d'au moins un segment de trajectoire dans une matrice de l'espace des k,
la surveillance (102) de la respiration du sujet en mesurant un ou plusieurs paramètres respiratoires,
la détermination de façon prospective d'un moment où la respiration correspondra à un état respiratoire de référence, et la commande d'au moins un paramètre d'acquisition du ou de chaque segment de trajectoire de sorte que ledit moment où la respiration correspondra à un état respiratoire de référence détermine le moment d'échantillonnage du point le plus proche du segment de trajectoire de l'espace des k par rapport au centre de la matrice de l'espace des k en utilisant le au moins un paramètre respiratoire surveillé,
**caractérisé en ce que** le procédé comprend en outre la détermination (103) d'un moment où le point le plus proche du segment de trajectoire de l'espace des k par rapport au centre de la matrice de l'espace des k a été échantillonné lors de l'acquisition (101) des données d'imagerie par résonance magnétique,
la détermination (104) du au moins un paramètre respiratoire pour ledit moment en utilisant ladite surveillance (102) de la respiration, et
la comparaison rétrospective du au moins un paramètre respiratoire déterminé correspondant audit moment d'échantillonnage du point le plus proche sur le segment de trajectoire par rapport au centre de la matrice de l'espace des k à une valeur de référence prédéterminée correspondant à l'état respiratoire de référence, afin de déterminer un déphasage à partir de cette comparaison et d'appliquer le déphasage aux valeurs de l'espace des k obtenues pour le segment de trajectoire afin de compenser une translation dans l'espace réel due au mouvement respiratoire tel que déterminé à partir de ladite comparaison.

2. Système selon la revendication 1, dans lequel ledit processeur (14) est adapté pour utiliser le au moins un paramètre respiratoire surveillé afin de déterminer une pluralité de moments où la respiration correspondra à l'état respiratoire de référence pour acquérir respectivement une pluralité de segments de trajectoire de sorte que chaque moment déterminé détermine le moment d'échantillonnage du point le plus proche sur chaque segment de trajectoire de l'espace des k par rapport au centre de la matrice de l'espace des k.

3. Système selon la revendication 2, dans lequel ledit processeur (14) est adapté pour commander le au moins un paramètre d'acquisition pour chacun de ladite pluralité de segments de trajectoire, en tenant compte d'une fenêtre temporelle prédéterminée pour l'acquisition collective de la pluralité de segments de trajectoire, et pour appliquer de manière adaptative une technique d'échantillonnage clairsemé et/ou de détection compressée sur et/ou au-delà de la fenêtre temporelle prédéterminée.

4. Système selon l'une quelconque des revendications précédentes, comprenant un dispositif de stockage de données (16), dans lequel ledit processeur (14) est adapté pour récupérer des images précédemment acquises du sujet à partir du dispositif de stockage de données (16), dans lequel des informations respiratoires sont associées à chacune desdites images précédemment acquises, le processeur étant en outre adapté pour enregistrer les images précédemment acquises et modéliser des transformations obtenues par ledit enregistrement en fonction des informations respiratoires afin d'obtenir un modèle de mouvement et de déterminer le déphasage pour compenser une translation dans l'espace réel due au mouvement respiratoire à l'aide dudit modèle de mouvement.

5. Système selon l'une quelconque des revendications précédentes, comprenant une unité de reconstruction (15) pour reconstruire les données d'imagerie par résonance magnétique corrigées en phase en au moins une image tomographique.

6. Système selon la revendication 5, dans lequel ladite unité de reconstruction (15) est adaptée pour annoter l'image tomographique reconstruite avec un état respiratoire qui est déterminé par le au moins un paramètre respiratoire qui est déterminé pour correspondre audit moment d'échantillonnage du point le plus proche sur chaque segment de trajectoire de l'espace des k par rapport au centre de la matrice de l'espace des k, par ladite valeur de référence prédéterminée utilisée pour déterminer le déphasage afin d'ajuster les données d'imagerie par résonance magnétique utilisées pour la reconstruction.

7. Système selon la revendication 5 ou 6, dans lequel ladite unité de reconstruction (15) est adaptée pour sélectionner un sous-ensemble approprié des données d'imagerie par résonance magnétique de façon à remplir la matrice de l'espace des k avec les données d'imagerie par résonance magnétique sélectionnées pour la construction, dans lequel les segments de trajectoire de l'espace des k sont sélectionnés sur la base d'une relation de l'au moins un paramètre respiratoire déterminé pour chaque segment de trajectoire de l'espace des k par rapport à un état respiratoire de référence à reconstruire.

8. Système selon l'une quelconque des revendications 5 à 7, dans lequel ledit processeur (14) est adapté pour commander l'acquisition des données d'imagerie par résonance magnétique en acquérant de manière répétée un même segment de trajectoire de l'espace des k à différents points du cycle respiratoire selon une stratégie de suréchantillonnage, dans lequel ladite unité de reconstruction (15) est adaptée pour reconstruire des images pour différents états respiratoires par une sélection correspondante à partir de, par interpolation entre et/ou par pondération des données acquises pour ledit même segment de trajectoire de l'espace des k.

9. Système selon l'une quelconque des revendications précédentes, comprenant une sortie de retour (12) pour fournir un retour sensoriel au sujet pendant la séance d'imagerie par résonance magnétique afin de guider le comportement respiratoire du sujet vers un modèle respiratoire prédéterminé.

10. Système selon l'une quelconque des revendications précédentes, dans lequel les données de capteurs sont synchronisées avec les données d'imagerie par résonance magnétique.

11. Système selon l'une quelconque des revendications précédentes, dans lequel le processeur est configuré pour déterminer le moment où le point le plus proche du ou de chaque segment de trajectoire de l'espace des k par rapport au centre de la matrice de l'espace des k au moins partiellement à l'aide du au moins un paramètre d'acquisition.

12. Procédé (100) de surveillance respiratoire de la respiration d'un sujet au cours d'une séance d'imagerie par résonance magnétique, le procédé comprenant :
l'acquisition (101) de données d'imagerie par résonance magnétique comprenant des valeurs de l'espace des k le long d'au moins un segment de trajectoire dans une matrice de l'espace des k,
la surveillance (102) de la respiration du sujet en mesurant un ou plusieurs paramètres respiratoires,
la détermination de façon prospective d'un moment où la respiration correspondra à un état respiratoire de référence, et la commande d'au moins un paramètre d'acquisition du ou de chaque segment de trajectoire de sorte que ledit moment où la respiration correspondra à un état respiratoire de référence détermine le moment d'échantillonnage du point le plus proche du segment de trajectoire de l'espace des k par rapport au centre de la matrice de l'espace des k en utilisant le au moins un paramètre respiratoire surveillé,
**caractérisé en ce que** le procédé comprend en outre la détermination (103) d'un moment où le point le plus proche du segment de trajectoire de l'espace des k par rapport au centre de la matrice de l'espace des k a été échantillonné lors de l'acquisition (101) des données d'imagerie par résonance magnétique,
la détermination (104) du au moins un paramètre respiratoire pour ledit moment en utilisant ladite surveillance (102) de la respiration, et
la comparaison rétrospective du au moins un paramètre respiratoire déterminé correspondant audit moment d'échantillonnage du point le plus proche sur le segment de trajectoire par rapport au centre de la matrice de l'espace des k à une valeur de référence prédéterminée correspondant à l'état respiratoire de référence, afin de déterminer un déphasage à partir de cette comparaison et d'appliquer le déphasage aux valeurs de l'espace des k obtenues pour le segment de trajectoire afin de compenser une translation dans l'espace réel due au mouvement respiratoire tel que déterminé à partir de ladite comparaison.

13. Produit programme d'ordinateur comprenant des instructions pour amener le système d'imagerie par résonance magnétique selon la revendication 1 à exécuter les étapes du procédé selon la revendication 12.
